(19) Europäisches Patentamt
European Patent Office
Office européen des brevets

(11) EP 4 531 945 B1

(12) **EUROPEAN PATENT SPECIFICATION**

(45) Date of publication and mention of the grant of the patent:
**05.08.2026 Bulletin 2026/32**

(21) Application number: **23728049.0**

(22) Date of filing: **23.05.2023**

(51) International Patent Classification (IPC):
*A61L 29/08* (2006.01) *A61L 29/14* (2006.01)
*A61L 31/10* (2006.01) *A61L 31/14* (2006.01)

(52) Cooperative Patent Classification (CPC):
(C-Sets available)
**A61L 31/10; A61L 29/085; A61L 29/14; A61L 31/14;** A61L 2420/02 (Cont.)

(86) International application number:
**PCT/EP2023/063740**

(87) International publication number:
**WO 2023/227577 (30.11.2023 Gazette 2023/48)**

(54) **HYDROPHILIC COATINGS FOR VASCULAR MEDICAL PRODUCTS**

HYDROPHILE BESCHICHTUNGEN FÜR VASKULÄRE MEDIZINISCHE PRODUKTE

REVÊTEMENTS HYDROPHILES POUR PRODUITS MÉDICAUX VASCULAIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.05.2022 EP 22175573**
**13.04.2023 EP 23167774**

(43) Date of publication of application:
**09.04.2025 Bulletin 2025/15**

(73) Proprietor: **BIOTRONIK AG**
**8180 Bülach (CH)**

(72) Inventors:
- **SCHWITZER, Alwin**
**8180 Buelach (CH)**
- **WINKLER, Martin**
**8854 Galgenen (CH)**
- **SIEGMANN, Konstantin**
**8606 Greifensee (CH)**
- **MU, Linyu**
**49448 Lemfoerde (DE)**
- **INAUEN, Jan**
**8610 Uster (CH)**

(74) Representative: **WSL Patentanwälte Partnerschaft mbB**
**Kaiser-Friedrich-Ring 98**
**65185 Wiesbaden (DE)**

(56) References cited:
**US-A1- 2020 086 008 US-B2- 8 632 877**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 29/085, C08L 39/06;**
**A61L 31/10, C08L 39/06**

## Description

The present invention generally relates to hydrophilic coatings for surfaces of vascular medical products. Particularly, the invention relates to the use of specific hydrophilic copolymers that can be covalently bound to the surfaces of vascular medical products by UV-radiation. Furthermore, the invention relates to the use of coating compositions containing the specific copolymers, and a method for coating the surface of a vascular medical product to provide the coating for the vascular medical product.

It is generally known to coat vascular medical products, such as catheters, balloon parts, or pacemaker electrodes, with hydrophilic polymers. Vascular medical devices require a lubricious coating to facilitate insertion into blood vessels and minimize damage to them. Lubrication is achieved by a hydrophilic coating that absorbs water and thus becomes slippery. US 2020/086008 A1 discloses a device having a switchable wet-dry lubricating coating, which is covalently bound directly on the surface of the device and comprises at least one polymer A, at least one cross-linker, comprising a core and at least two reactive groups, and at least one lubricant. Perfluorophenyl azide (PFPA) is the preferred reactive group. However, the known systems are either expensive due to the chemicals used, or do not reliably meet the requirements for vascular implantable medical products, particularly providing a permanent coating on the surfaces with very low number of particles released, and/or low friction to blood vessels. It is further known that hydrophobic surfaces, such as polyolefins or polysiloxanes, are difficult to coat with hydrophilic compounds.

Therefore, there is a need to provide improved coatings for vascular medical products that overcome problems of the prior art. Particularly, the coatings should be hydrophilic and allow an easy to perform or automatic application. The coatings should also be applicable to surfaces that are difficult to coat, e.g., hydrophobic surfaces. Moreover, the coatings should lead to low friction to friction partners, e.g., blood vessels, and release of a very low number of particles.

This problem is solved by a method for coating a surface of a vascular medical product with a coating composition of claim 1, by a vascular medical product of claim 7 and the use of claim 10. Preferred embodiments are described in the dependent claims.

A method for coating a surface of a vascular medical product or at least one part thereof with a coating composition is described, wherein the coating is applied to the vascular medical product, and wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer having at least

1) repeating units of vinylpyrrolidone, and
2) at least one perfluorophenyl azide moiety,

d) 0 to 1 weight% of a multifunctional UV-sensitive lower-molecular phenyl azide compound having an UV-absorption in the range of 200 to 300 nm,

wherein the components (a) to (d) are dissolved in 50 to 90 volume% of alcohol, preferably ethanol and/or 2-propanol, and 10 to 50 volume% of water
and optionally the applied copolymeric coating is dried and/or irradiated by UV irradiation.

Thus, a method for coating a surface of a vascular medical product or at least one part thereof with a copolymeric coating according to the following chemical process is described:

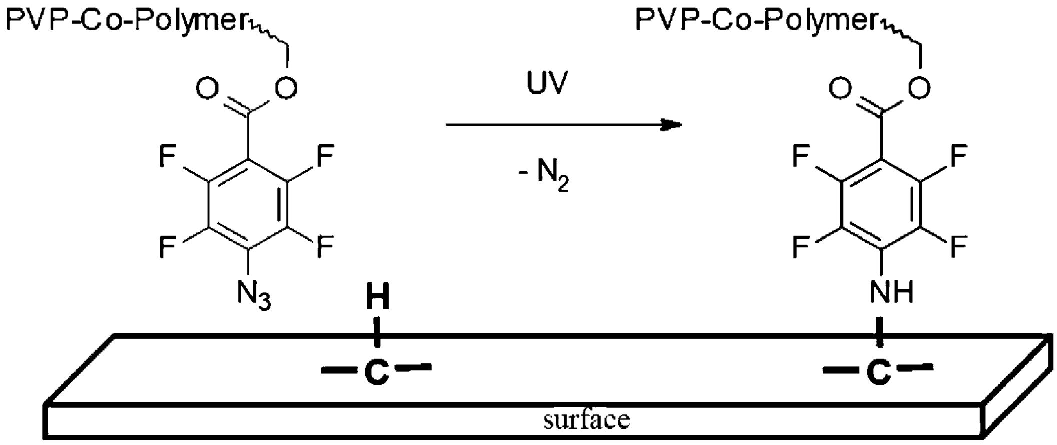


wherein the at least one copolymer comprises

a) repeating units of vinylpyrrolidone, and
b) at least one perfluorophenyl azide moiety,

wherein the at least one perfluorophenyl azide moiety is inserted in a chemical C-H bond of the surface of the vascular medical product (e.g. an interventional catheter) or the at least one part thereof, preferably by irradiating the copolymeric coating and the surface of the vascular medical product or the at least one part thereof with UV irradiation.

The vascular medical product, preferably obtained by the method according to the present invention, comprises a coating composition, wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer having at least

 1) repeating units of vinylpyrrolidone, and
 2) at least one perfluorophenyl azide moiety, and

d) 0 to 1 weight% of a multifunctional UV-sensitive lower-molecular phenyl azide compound having an UV-absorption in the range of 200 to 300 nm.

The copolymeric coating can be applied to the surface of the vascular medical product or to the at least one part thereof by dip coating, spray coating, roller coating, printing, painting, or by means of a doctor blade, doctor roller, or Langmuir-Blodgett films, then the applied composition is dried and irradiated by UV light. The surface of the vascular medical product or the at least one part thereof may be dipped into the coating composition and drawn out from the coating composition at a defined velocity to obtain a defined coating thickness. The method may be carried out in automated manner.

The surface of the vascular medical product or the at least one part thereof may comprise a polymer selected from a thermoplastic elastomer, polyamide, polyetherblockamides, thermoplastic polyurethanes (TPU), polyester, polyester-based elastomers, polyolefins, vinylpolymers, polysiloxanes, and combinations thereof, preferably selected from a thermoplastic elastomer, a polyamide, a polyetherblockamide, thermoplastic polyurethanes (TPU), thermoplastic elastomer (TPE) such as a thermoplastic elastomer comprising polyamide and polyether blocks, preferably a polyether block amide (e.g. Pebax or Vestamid).

Thus, the obtained vascular medical product is coated with a (hydrophilic) copolymeric coating comprising at least one of the following copolymers or consisting of the following copolymer:

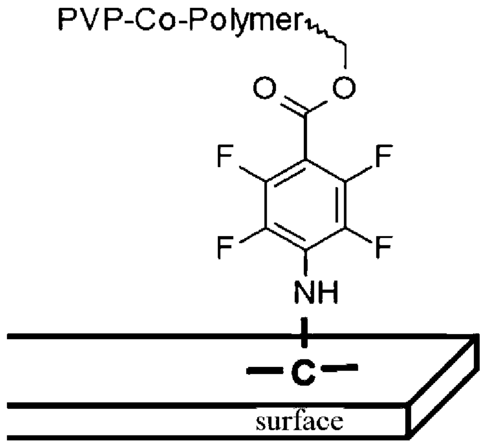

Therefore, a vascular medical product or at least one part thereof comprising a copolymeric coating is described, wherein the copolymeric coating is bound to a surface of the vascular medical product and comprises at least one of the following copolymers or consists of the following copolymer:

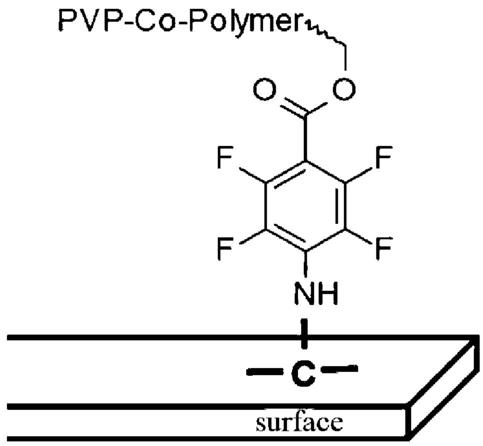

The above described (hydrophilic) copolymeric coating that absorbs water and thus becomes slippery enables a lubrication within the human or animal body (e.g. in a blood vessel). The (hydrophilic) copolymeric coating is mechanically resistant. The (hydrophilic) copolymeric coating is chemically bound to the surface of the vascular medical product or the at least one part thereof. Thus, no particles are released from the surface, therefore the (hydrophilic) copolymeric coating poses a lower risk for thrombosis, embolism, and arterial blockage. The (hydrophilic) copolymeric coating show very good sliding and abrasion resistance compared to prior art products.

Water-soluble polyvinylpyrrolidone (PVP) serves for the hydrophilic properties. Mono- as well as multi-functional perfluorophenyl azide (PFPA) copolymers can be used for crosslinking and chemical bonding to the vascular medical product or the part thereof, e.g. an (interventional) catheter surface.

In one embodiment the vascular medical product may comprise a (hydrophilic) copolymeric coating, wherein the (hydrophilic) copolymeric coating comprises or consists of the following copolymer:

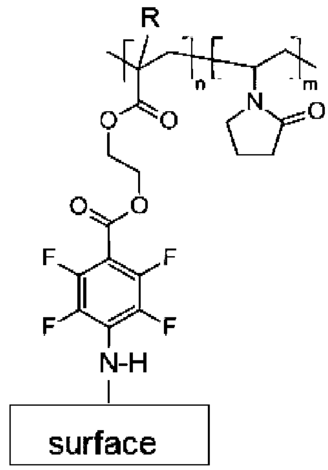

,

wherein R is H or CH3 and wherein n is an integer 1 or 2, and m is an integer in the range of from 15 to 35, preferably wherein n is an integer 1 and m is an integer in the range of from 20 to 30.

In another embodiment the vascular medical product may comprise a (hydrophilic) copolymeric coating, wherein the (hydrophilic) copolymeric coating comprises or consists of the following copolymer:

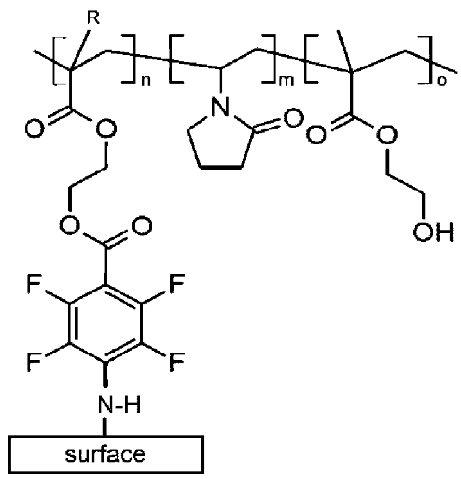

wherein R is H or CH3 and wherein n is 1 or 2, m is 15 to 25, and o is 5 to 15, preferably wherein n is 1, m is 20, and o is 10.

In a further embodiment the vascular medical product may comprise a (hydrophilic) copolymeric coating, wherein the (hydrophilic) copolymeric coating comprises or consists of the following copolymer:

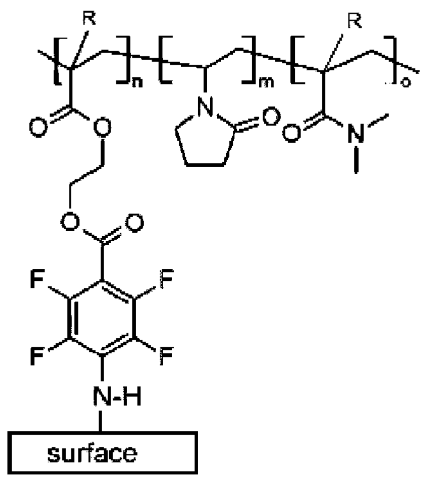

, wherein R is H or CH3 and wherein n is 1 or 2, m is 15 to 25, and o is 5 to 15, preferably wherein n is 1, m is 20, and o is 1 to 10.

The (hydrophilic) copolymeric coating may be (covalently) bound to a surface of the vascular medical product, preferably via a carbon nitrogen (C-N) bond.

The vascular medical product is a medical product or a part thereof, which is intended to be implanted or introduced into a vessel, particularly in the fields of electrophysiology, heart-rhythm management, neurostimulation, vascular and endovascular intervention, intervention in and at the heart. The vascular medical product may be selected from coronary or peripheral catheters or parts thereof, CRM devices or parts, balloon parts, or pacemaker electrodes. The surface of the vascular medical product may comprise or may be made of a polymer selected from a thermoplastic elastomer, polyamide, polyetherblockamides, thermoplastic polyurethanes (TPU), polyester, polyester-based elastomers, polyolefins, vinylpolymers, polysiloxanes, and combinations thereof, preferably selected from a, a polyamide, a polyetherblockamide, thermoplastic polyurethanes (TPU), thermoplastic elastomer (TPE) such as a thermoplastic elastomer comprising polyamide and polyether blocks, preferably a polyether block amide (e.g. Pebax or Vestamid).

The (hydrophilic) copolymeric coating can be applied to the vascular medical product or a part thereof, e.g. (interventional) catheter (hose), via a dip coating process.

Also a vascular medical product, comprising a (hydrophilic) copolymeric coating is disclosed wherein the (hydrophilic) copolymeric coating comprises or consists of the following segments:

- an azide,
- an aromatic moiety being bound to the azide,
- an ester being bound to the aromatic moiety,
- at least one hydrophilic repeating unit being bound to the ester.

In one embodiment the vascular medical product comprises a (hydrophilic) copolymeric coating wherein the (hydrophilic) copolymeric coating comprises or consists of the following segments:

- an azide,
- a perfluorophenyl moiety being bound to the azide,
- an ester being bound to the perfluorophenyl moiety,
- an acrylate or methacrylat or repeating units of an acrylate or methacrylate being bound to the ester,
- repeating units of vinylpyrrolidone being bound to the acrylate or methacrylat or repeating units of an acrylate or methacrylate, and optionally
- repeating units being bound to the vinylpyrrolidone repeating units and wherein the repeating units are selected from of 2-hydroxyethyl methacrylate (HEMA), N,N-dimethyl (meth)acrylate and N,N-dimethylacrylamide (DMA).

Due to its hydrophobic nature the phenylazide can be bound preferably to hydrophobic surfaces, e.g. that of a vascular medical product. The aromatic moiety, preferably the perfluorophenyl moiety, is configured to absorb UV irradiation and to activate the azide. The azide is a reactive group, which upon activation, preferably UV-irradiation, splits off dinitrogen ($N_2$) forming a highly reactive nitrene intermediate that is able to insert into a C-H bond of a vascular medical product surface. Thus, the azide or the copolymer comprising an azide group becomes covalently bond to the surface of the vascular medical product. The (poly)vinylpyrrolidone provides hydrophilic properties.

Furthermore, the present invention meets this need at least in part by providing use of a UV-sensitive copolymer for coating a surface of a vascular medical product, wherein the UV-sensitive copolymer comprises at least repeating units of (a) vinylpyrrolidone, and (b) perfluorophenylazide, wherein the copolymer has a statistical distribution of the repeating units (a) and (b) and has a molecular weight of 5,000 to 50,000 g/mol. The invention further relates to use of a coating composition for coating a surface of a vascular medical product, wherein the coating composition contains the copolymer, a method for coating the surface of a vascular medical product, and the coated vascular medical products obtained.

The vascular medical product may relate to a medical product or a part thereof which may be or are (to be) inserted or introduced into a vessel, e.g. but not limited in the fields of electro-physiology, cardiac rhythm management, neurostimulation, vascular or endovascular intervention as well as cardiac intervention.

The surface of the vascular medical product comprises a polymer selected from a thermoplastic elastomer (TPE), polyamide, polyetherblockamides, thermoplastic polyurethanes (TPU), polyester, polyester-based elastomers, polyolefins, vinylpolymers, polysiloxanes, and combinations thereof. The surface of the vascular medical product preferably comprises a polymer selected from a thermoplastic elastomer, a polyamide, a polyetherblockamide, thermoplastic polyurethanes (TPU), thermoplastic elastomer (TPE) such as a thermoplastic elastomer comprising polyamide and polyether blocks, preferably a polyether block amide (e.g. Pebax or Vestamid). A polyether block amide is a block copolymer obtained by polycondensation of a carboxylic acid polyamide (PA6, PA11, or PA12) with an alcohol termination polyether (Polytetramethylene glycol (PTMG) or PEG).

Herein, the term "(meth)acrylate" is used to designate "acrylate" or "methacrylate", as commonly used in the art.

Herein, the term "slippery" in the context of a surface of a vascular medical product has the meaning that the vascular medical product can be inserted into a body without leading to injuries and/or causing pain or discomfort to the patient.

Embodiments of the vascular medical product and of the use of a coating composition for a method of coating a surface of a vascular medical product are provided below:

Embodiment 1. A vascular medical product, comprising a (hydrophilic) coating, wherein the (hydrophilic) coating comprises at least one copolymer having at least

a) repeating units of vinylpyrrolidone, and
b) at least one perfluorophenyl azide moiety, wherein a repeating unit of vinylpyrrolidone comprises more monomer units than a repeating unit of perfluorophenyl azide moiety.

Embodiment 2. The vascular medical product according to embodiment 1, wherein the copolymer comprises 1 to 50 mol% of the repeating unit a), and 0.1 to 5 mol% of the repeating unit b).

Embodiment 3. The vascular medical product according to one of the embodiments 1 or 2, wherein the copolymer has a statistical distribution of the repeating units a) and b).

Embodiment 4. The vascular medical product according to any one of the embodiments 1 to 3, wherein the copolymer further comprises at least one repeating unit c) selected from of 2-hydroxyethyl methacrylate (HEMA), N,N-dimethyl (meth)acrylate, and N,N-dimethylacrylamide (DMA).

Embodiment 5. The vascular medical product according to embodiment 4, wherein the copolymer comprises 1 to 20 mol% of the repeating unit c), preferably in a statistical distribution.

Embodiment 6. The vascular medical product according to any one of the preceding embodiments, wherein the copolymer has a molecular weight of 5,000 to 50,000 g/mol.

Embodiment 7. The vascular medical product according to any one of the preceding embodiments, wherein the (hydrophilic) copolymeric coating is (covalently) bound to a surface of the vascular medical product, preferably via a carbon nitrogen (C-N) bond.

Embodiment 8. The vascular medical product according to any one of the preceding embodiments, wherein the vascular medical product is a medical product or part thereof, which is intended to be implanted or introduced into a vessel, particularly in the fields of electrophysiology, heart-rhythm management, neurostimulation, vascular and endovascular intervention, intervention in and at the heart.

Embodiment 9. The vascular medical product according to any one of the preceding embodiments, wherein the vascular medical product is selected from coronary or peripheral catheters or parts thereof, CRM devices or parts, balloon parts, or pacemaker electrodes.

Embodiment 10. The vascular medical product according to any one of the preceding embodiments, wherein the surface of the vascular medical product comprises or is made of a polymer selected from a thermoplastic elastomer, polyamide, polyetherblockamides, thermoplastic polyurethanes (TPU), polyester, polyester-based elastomers, polyolefins, vinylpolymers, polysiloxanes, and combinations thereof, preferably selected from a, a polyamide, a polyetherblockamide, thermoplastic polyurethanes (TPU), thermoplastic elastomer (TPE) such as a thermoplastic elastomer comprising polyamide and polyether blocks, preferably a polyether block amide (e.g. Pebax or Vestamid).

Embodiment 11. Use of a copolymeric coating for coating a surface of a vascular medical product, wherein the copolymeric coating comprises or consists of a copolymer having at least

a) repeating units of vinylpyrrolidone, and
b) at least one perfluorophenyl azide moiety, and optionally
c) repeating units selected from of 2-hydroxyethyl methacrylate, N,N-dimethyl(meth)acrylate, and N,N-dimethylacrylamide.

Embodiment 12. Use of a (UV-sensitive, hydrophilic) copolymer for coating a surface of a vascular medical product, wherein the (UV-sensitive, hydrophilic) copolymer comprises at least repeating units of

(a) vinylpyrrolidone, and
(b) perfluorophenylazide,

wherein a repeating unit of vinylpyrrolidone comprises more monomer units than a repeating unit of perfluorophenylazide;
wherein the copolymer has a statistical distribution of the repeating units (a) and (b) and has a molecular weight of 5,000 to 50,000 g/mol.

Embodiment 13. The use according to embodiment 11 or 12, wherein the copolymer comprises 1 to 50 mol% of the repeating unit (a), and 0.1 to 5 mol% of the repeating unit (b).

Embodiment 14. The use according to embodiment 11, wherein the copolymer comprises 1 to 20 mol% of the repeating unit (c), preferably in a statistical distribution.

Embodiment 15. Use of a (UV-sensitive, hydrophilic) copolymer for coating a surface of a vascular medical product, wherein the copolymer comprises the following repeating units:

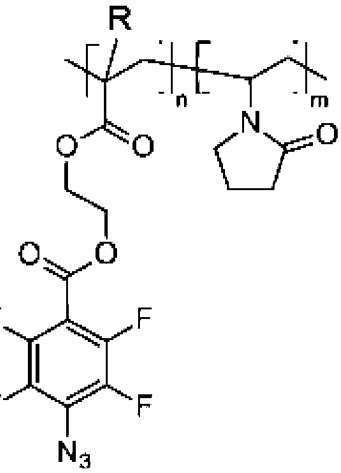

wherein n is an integer 1 or 2, m is an integer in the range of from 15 to 30 and wherein R is H or $CH_3$.

Embodiment 16. Use of a (UV-sensitive, hydrophilic) copolymer for coating a surface of a vascular medical product, wherein the copolymer comprises the following repeating units:

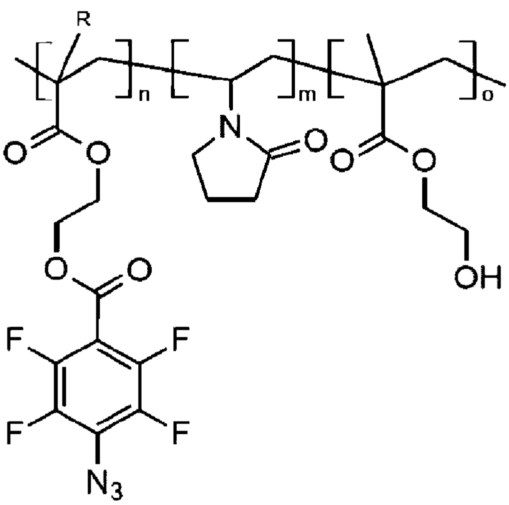

wherein n is 1, m is 20, and o is 10 and wherein R is H or $CH_3$;
or

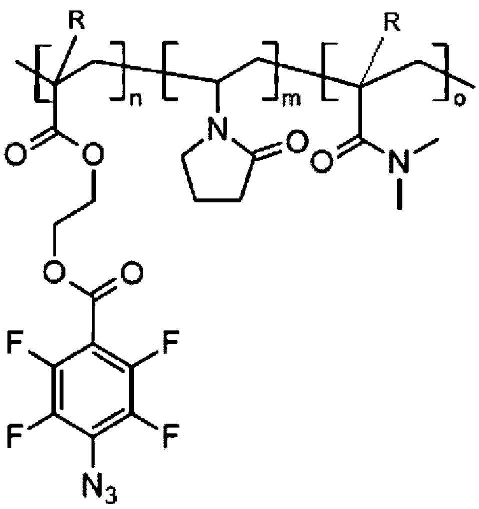

wherein n is 1, m is 20, and o is 10 and wherein R is H or $CH_3$.

Embodiment 17. Use of a coating composition for coating a surface of a vascular medical product, wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer as defined in any one of the preceding claims examples, and
d) 0 to 1 weight% of a multifunctional UV-sensitive lower-molecular phenyl azide compound having an UV-absorption in the range of 200 to 300 nm,

wherein the components (a) to (d) are dissolved in 50 to 90 volume% of alcohol, preferably ethanol and/or 2-propanol, and 10 to 50 volume% of water.

Embodiment 18. The use according to embodiment 17, wherein the phenyl azide compound d) is a triazide, polyethyleneglycol-based bisazide, diol bisazide, and/or an ionic bisazide.

Subsequently, embodiments of the invention shall be described in detail with reference to the drawings. It is to be noted that the embodiments are not limiting for the invention, but merely represent illustrative examples. The figures show:

- Fig. 1 shows a procedure for adding the perfluorophenyl (meth)acrylate into the polymerization vessel via a syringe pump;
- Fig. 2 shows the molecular weight distribution of an exemplary copolymer by GPC;
- Fig. 3 shows the $^{19}$F-NMR spectrum of an exemplary copolymer;
- Fig. 4 shows the $^{1}$H-NMR spectrum of an exemplary copolymer;
- Fig. 5 shows $^{1}$H-NMR, $^{19}$F-NMR and FT-IR spectra as well as molecular weight distribution for another exemplary copolymer; and
- Fig. 6 shows schematically the binding of the copolymer to a surface.

The copolymer used according to the invention comprises at least repeating units of vinylpyrrolidone (VP), and repeating units of perfluorophenylazide (PFPA), preferably perfluorobenzoyl (meth)acrylate azide. A repeating unit of vinylpyrrolidone comprises more monomer units than a repeating unit of perfluorophenylazide comprises monomer (PFPA) units. Preferably, a repeating unit of vinylpyrrolidone (VP) comprises 15 to 35 VP units, more preferably 20 to 30 VP units. Preferably, a repeating unit of PFPA comprises 1 to 3 PFPA units, more preferably 1 or 2 PFPA units, most preferably 1 PFPA unit. An exemplary copolymer comprises the following repeating units:

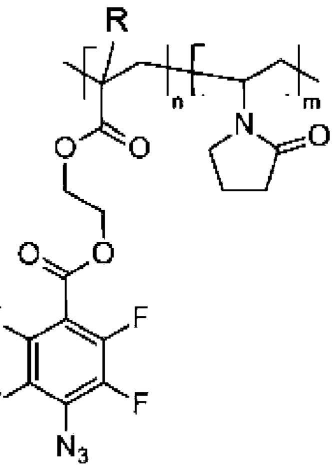

, wherein n is an integer of 1, 2 or 3, preferably 1 or 2, and wherein m is an integer in the range of from 15 to 35 and wherein R is H or $CH_3$.

The polyvinylpyrrolidone repeating units impart hydrophilic and slippery properties to the coatings obtained by using the copolymer. The perfluorophenylazide units enable to covalently bind or attach the copolymer to surfaces, particularly to polymeric or siloxane materials, via the azide group which can be activated by UV-radiation. The reaction mechanism of the activation by the azide group is known from the article of Siegmann, K., Inauen, J., Villamaina, D. and Winkler, M., "Photografting of perfluoroalkanes onto polyethylene surfaces via azide/nitrene chemistry." Applied Surface Science, 396 672-680 (2017), and the article of Siegmann, K., Inauen, J., Sterchi, R. and Winkler, M., "Spectroscopy on photografted polyethylene surfaces using a perfluorophenyl azide: Evidence for covalent attachment." Surface and Interface Analysis, 50 (2) 205-211 (2018). The mechanism is shown hereinafter, wherein R designates the corresponding residue of the perfluorophenylazide unit:

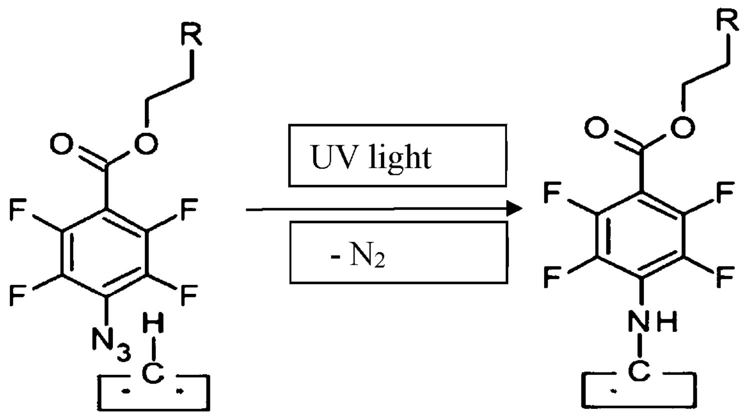


Upon UV activation, preferably at a wavelength between 200 and 350 nm, more preferably between 240 and 320 nm, the azide group is converted to molecular nitrogen which splits off, and a highly reactive nitrene, which undergoes a very fast non-specific insertion reaction into a C-X bond of a substrate, for example, a C-H bond. Due to the presence of several PFPA repeating groups and thus several azide groups in a copolymer, the copolymer can be covalently bound and thus permanently attached at these several positions to the substrate.

The copolymer has a statistical distribution of the VP repeating units (a) and PFPA repeating units (b) and has a molecular weight of 5,000 to 50,000 g/mol, preferably of 10,000 to 45,000 g/mol.

In preferred embodiments, the copolymer comprises further hydrophilic repeating units, for example, a repeating unit (c) derived from 2-hydroxyethyl methacrylate (HEMA), N,N-dimethyl (meth)acrylate, and N,N-dimethylacrylamide (DMA). The additional repeating units further improve the hydrophilic and slippery performance of coatings obtained by using the copolymer.

An exemplary copolymer can comprise the following repeating units:

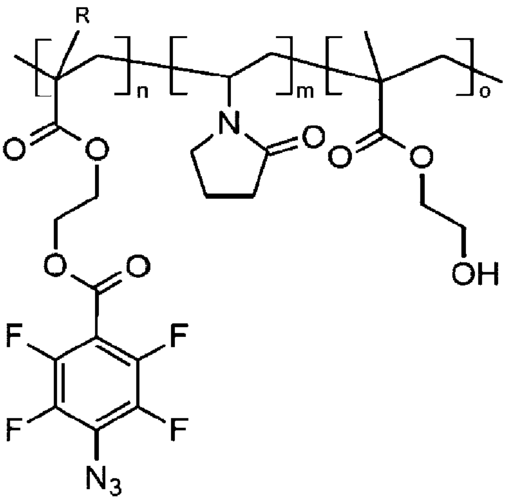

,

wherein, for example, n is 1, m is 15 to 25, preferably 20, and o is 5 to 15, preferably 10; and wherein R is H or $CH_3$.

Another exemplary copolymer can comprise the following repeating units:

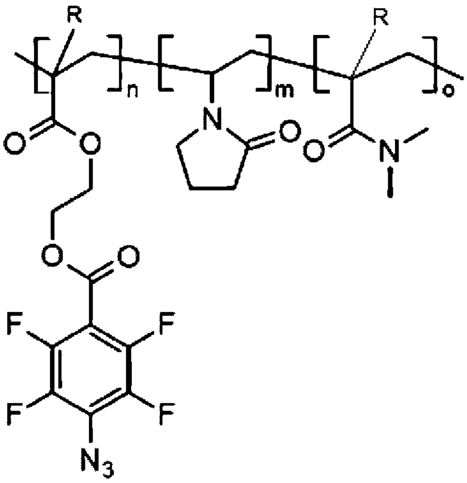

-

wherein, for example, n is 1, m is 15 to 25, preferably 20, and o is 5 to 15, preferably 10, and wherein R is H or $CH_3$.

In preferred embodiments, the copolymer used according to the invention comprises 1 to 50 mol% of vinylpyrrolidone repeating unit (a), preferably 5 to 45 mol% of vinylpyrrolidone repeating unit (a), and 0.1 to 5 mol% of PFPA repeating unit (b), preferably 1 to 5 mol% of PFPA repeating unit (b). In exemplary copolymers that comprise further repeating units (c) as described above, the copolymer comprises 1 to 20 mol% of the repeating unit (c). In further preferred embodiments, the repeating units (a), (b) and (c) are statistically distributed.

The copolymer used according to the invention can be prepared by a process comprising at least the following steps:

(a) esterification of perfluorobenzoyl chloride with 2-hydroxyethyl (meth)acrylate;
(b) reacting the product obtained in step (a) with sodium azide, to obtain 4-azidoperfluorophenyl acrylate; and
(c) copolymerizing the 4-azidoperfluorophenyl azide acrylate of step (b) with vinylpyrrolidone using a radical initiator.

The esterification step (a) can be carried out as described in the following reaction scheme:

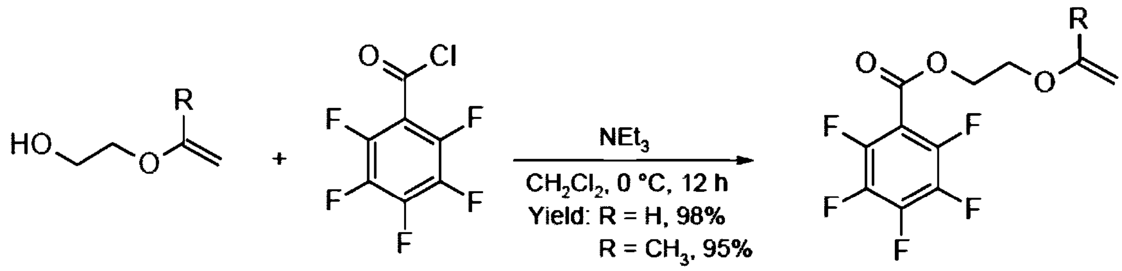


As shown, perfluorobenzoyl chloride is reacted with a 2-hydroxyethyl (meth)acrylate with R designating H or methyl, in triethylamine. In exemplary embodiments, the reaction is carried out in dichloromethane at about 0°C and for about 12 hours. Exemplary yields are, 98% for R = H, and 95% for R = methyl. The compounds obtained can be characterized by e.g., FT-IR, $^{1}$H and $^{19}$F-NMR spectroscopy.

Compounds preferably obtained after step (a) are shown hereinafter (left: methacrylate; right: acrylate):

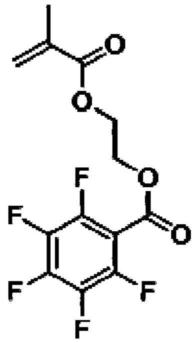

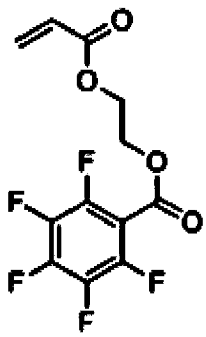

It is also possible to use an acrylamide or vinylether, instead of 2-hydroxyethyl (meth)acrylate, resulting in the following compounds (left: acrylamide; right: vinylether):

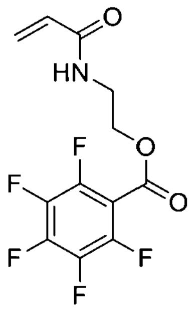

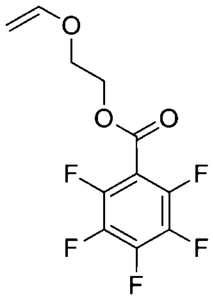

After step (a), the product is reacted with an azide, preferably sodium azide. The reaction can be carried out under suitable conditions, e.g., at room temperature and for 24 hours. In step (b), the azide group is introduced by substituting a F atom of the perfluorophenyl ring.

In step (c), the product obtained in step (b) is polymerized with vinylpyrrolidone, wherein vinylpyrrolidone is used in excess of the azidoperfluorophenyl acrylate. The copolymerization is initiated with a radical polymerization initiator, for example, AIBN or dibenzoyl peroxide (BPO). The copolymerization is carried out in a manner known in the art.

An exemplary method with the steps (a) to (c) is shown in the scheme hereinafter (esterification, azide substitution, polymerization):

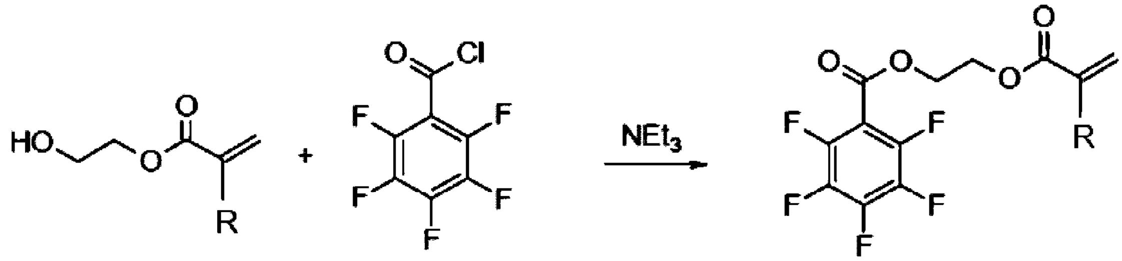


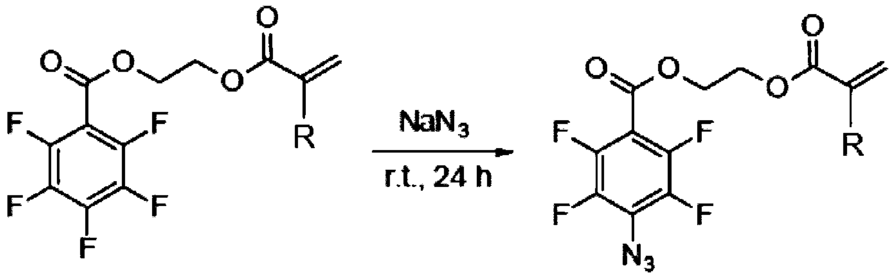


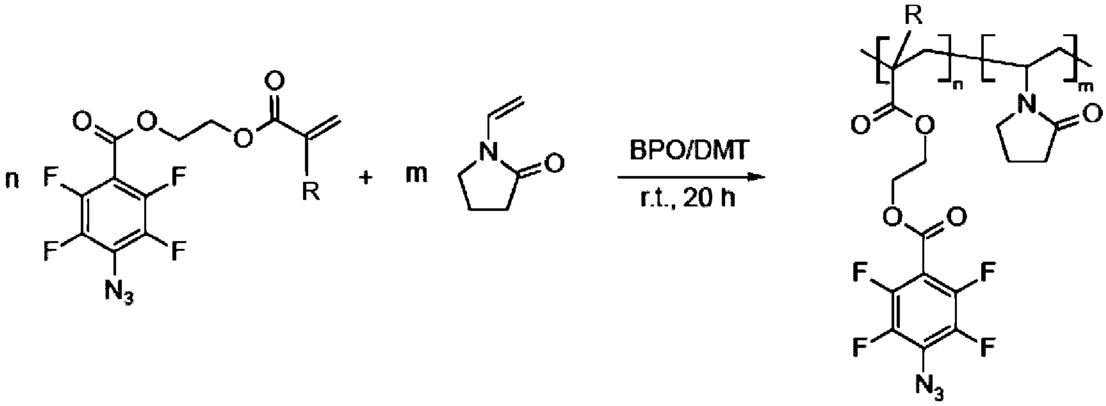


,

wherein n is an integer of 1, 2 or 3, preferably 1 or 2, most preferably 1 and designates the number of perfluorophenylazide units, specifically the perfluorobenzoyl (meth)acrylate azide units, and m is an integer in the range of from 15 to 35, preferably 20 to 30, which designates the number of vinylpyrrolidone units, and wherein R is H or $CH_3$.

The polymerization can be optimized by adding the azidoperfluorophenyl (meth)acrylate, for example, via a syringe pump and/or over a period of 4 to 8 hours. The addition over a period of 4 to 8 hours of the acrylate promotes the formation of a copolymer with statistic distribution of the repeating units, due to the different copolymerization parameters of the monomers used, such as their different reaction rates. Contratry, a fast addition (less than 4 hours) and/or an addition without the syringe pump of the acrylate promotes the formation of a blockcopolymers. As an example, for methacrylate r = approx. 3.9, and for vinylpyrrolidone r = 0.004. Figure 1 shows an exemplary procedure. According to Figure 1, the azidoperfluorophenyl (meth)acrylate is slowly added (via the syringe pump), e.g. over a time period of 4 to 8 hours, into the polymerization vessel containing vinylpyrrolidone and an initiator (e.g. AIBN as shown).

For an exemplary copolymer of the invention, Figure 2 shows the molecular weight distribution by means of GPC (gel permeation chromatography) in units of normalized intensity vs. molar mass (g/mol). A unimodal distribution is obtained.

Figures 3 and 4 show $^{19}F$-NMR and $^{1}H$-NMR spectra. Figure 4 also shows how the ratio of the two repeating units to each other or the two parameters m and n can be determined from the $^{1}H$-NMR spectrum due to H atoms at certain positions.

The $^{1}H$-NMR, $^{19}F$-NMR and FT-IR spectra (transmission vs. wavenumber ($cm^{-1}$)) as well as molecular weight distribution for another exemplary copolymer are shown in Figure 5.

In exemplary embodiments, the copolymerization is carried out using dibenzoyl peroxide (BPO) in N,N-Dimethyl-p-toluidine (DMT) at room temperature. A suitable reaction scheme is shown hereinafter:

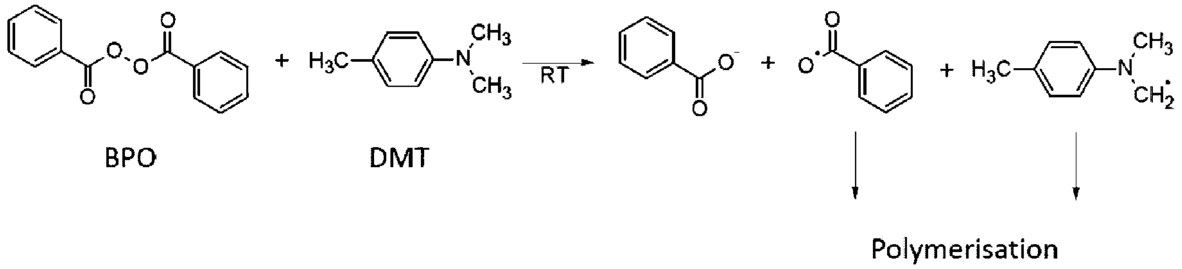


Alternatively, the copolymerization can be carried out using reversible-addition-fragmentation chain-transfer (RAFT) polymerization in the presence of BPO, DMT and benzyl 1H-imidazole-1-carbodithioate (BICDT). A suitable reaction is shown hereinafter:

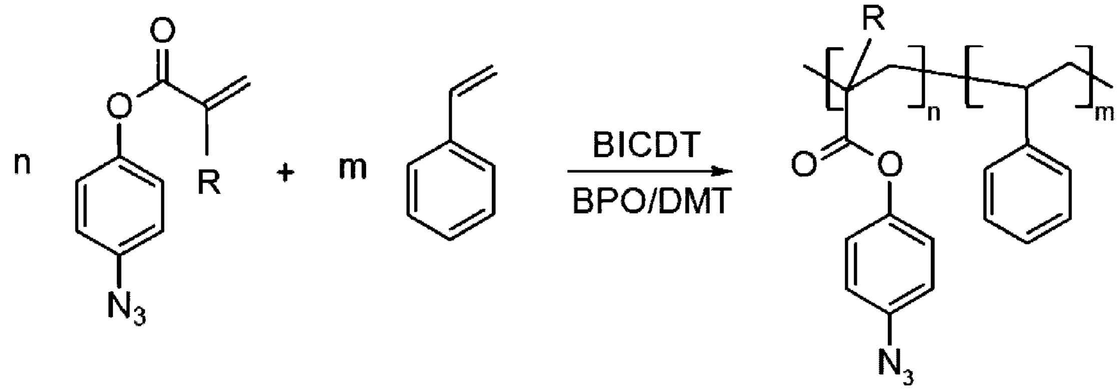


wherein R is H or $CH_3$.

It is also possible to change in the described preparation method with the steps (a), (b) and (c), the order of the steps (b) and (c), that is, the copolymerization is carried out before the azide substitution. An exemplary reaction route is shown hereinafter (top: free radical polymerization; bottom: azide substitution):

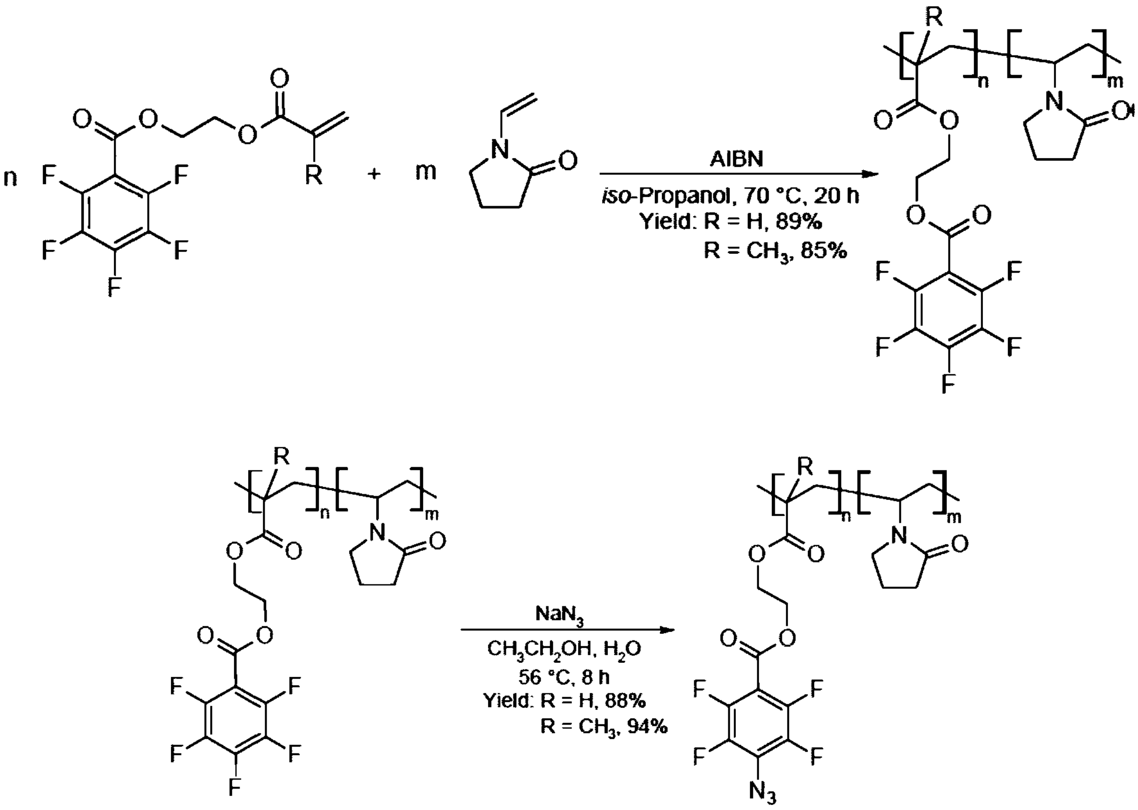

In further preferred embodiments of the invention, the copolymerization in step (c) is carried out in the presence of further monomers, for example, 2-hydroxyethyl methacrylate (HEMA), N,N-dimethyl (meth)acrylate, and N,N-dimethylacrylamide (DMA). Exemplary repeating units are shown hereinafter:

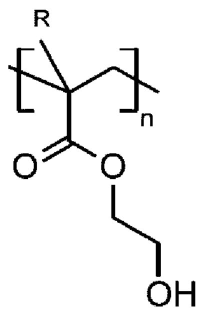
PHEMA

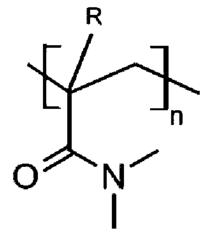
PDMA,

wherein R is H or $CH_3$. PHEMA denotes poly(2-hydroxyethyl methacrylate) (HEMA) and PDMA denotes poly(N,N-dimethylacrylamide).

Exemplary copolymers comprise at least one of the following repeating units:

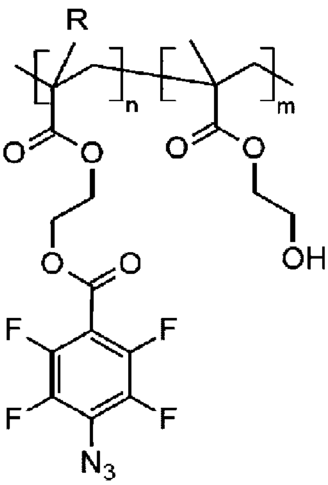

wherein R is H or $CH_3$ and wherein, for example, the molecular weight $M_n$ is 33,000 g/molthe ratio m:n is 20:1 and preferably the PFPA content is 5 mol%. Preferably, the copoylmer is PHEMA-co-PFPA having a molecular weight $M_n$ of 33,000 g/mol; the ratio m:n is 20:1; the PFPA content is 5 mol%. The solvent is preferably ethanol/water in a ratio of 1:1; and the concentration of the copolymer is 20 weight%.

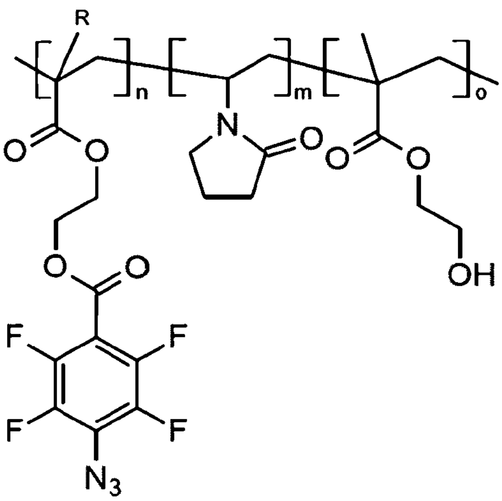

wherein R is H or $CH_3$ and wherein, for example, the molecular weight $M_n$ is approx. 50,000 g/mol; the ratio m:o:n is 20: 10:1 and wherein preferably the PFPA content is 3 mol% and the PDMA content is 32 mol%. Preferably, the copolymer is PVP-co-PHEMA-co-PFPA wherein the PFPA content is 3 mol%; and the PDMA content is 32 mol%.

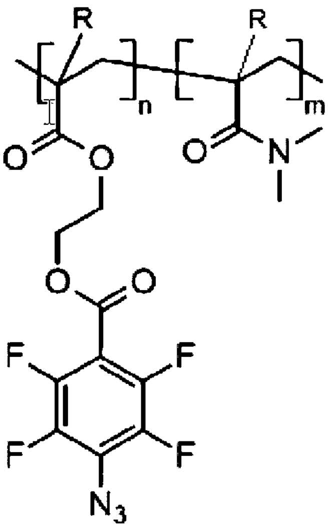

wherein, for example, the molecular weight $M_n$ is 14,000 g/mol; the ratio m:n is 20:1 wherein preferably the PFPA content is 5 mol%. Preferably, the copolymer is PDMA-co-PFPA wherein the PFPA content is 5 mol%. The solvent is preferably acetone/water in a ratio of 1:1; and the copolymer concentration is 20 weight%.

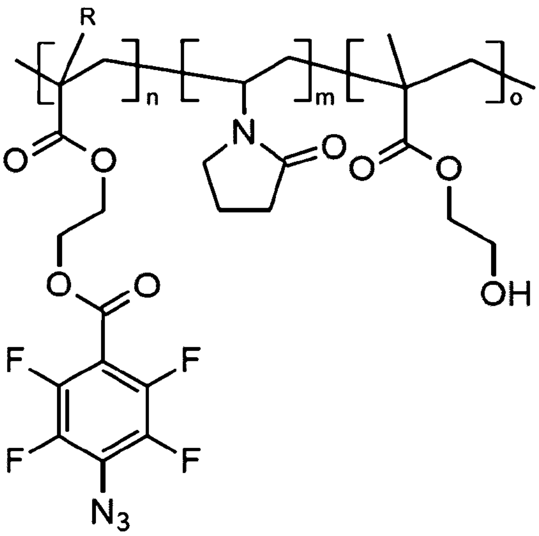

wherein R is H or $CH_3$ and wherein, for example, the molecular weight $M_n$, in g/mol, is 12,000 to 30,000; the ratio m:o:n is 20: 1:1 to 20:10:1 and preferably the PFPA content is 3 mol% to 5 mol% and preferably the PDMA content is 5 mol% to 32 mol%. Preferably, the copolymer is PVP-co-PDMA-co-PFPA having a molecular weight Mn, in g/mol, of 30,000 or 12,000; the ratio m:o:n is 20: 1:1 or 20:10:1; and preferably the PFPA content is 5 mol% or 3 mol%; and the PDMA content is 5 mol% or 32 mol%. The solvent is preferably acetone/water in a ratio of 1:1; and the copolymer concentration is 20 weight%.

The present invention provides use of a coating composition for coating a surface of a vascular medical product, wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer of the invention, and
d) 0 to 1 weight% of a multifunctional UV-sensitive lower-molecular phenyl azide compound having an UV-absorption, preferably in the range of 200 to 300 nm.

The components a) to d) are dissolved in aqueous alcohol, preferably in 50 to 90 volume% of alcohol, and 10 to 50 volume% of water. The alcohol is preferably ethanol and/or 2-propanol.

The copolymer c) is a copolymer as described above.

The phenyl azide compound d) is preferably a triazide, polyethyleneglycol-based bisazide, diol bisazide, and/or an ionic bisazide. Exemplary multifunctional phenyl azide compounds are shown hereinafter (in this order: triazide, PEG-bisazide, diol-bisazide, ionic bisazide):

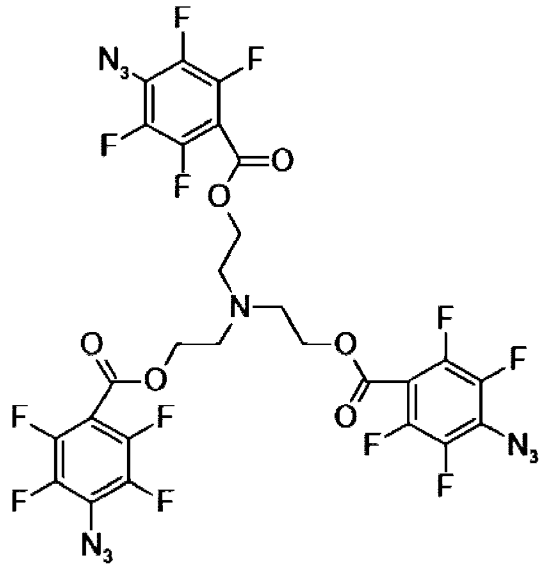

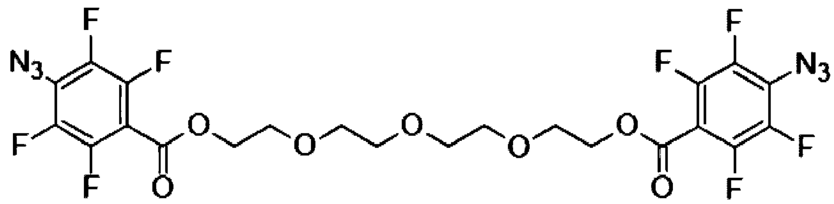

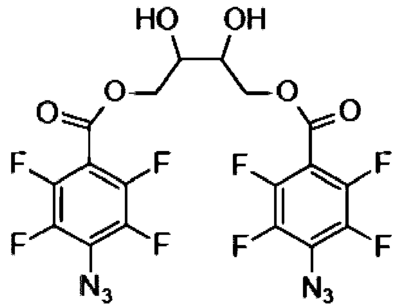

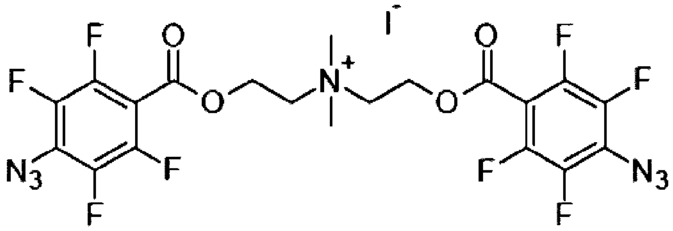

The present invention further provides a method for coating a surface of a vascular medical product. In this method, a coating composition of the invention or a coating composition as described above, is applied to the surface of a vascular medical product by a procedure known in the art. Suitable application methods are dip coating, spray coating,

roller coating, printing, or painting. The coating composition also can be applied by means of a doctor blade, a doctor roller, or Langmuir-Blodgett films. A preferred method is dip-coating. After its application on the surface, the applied composition is dried. Thereafter, the composition is irradiated by UV light. The UV light has preferably a wavelength in the range of from 200 to 350 nm, more preferably from 240 and 320 nm. A suitable wavelength is, for example, 254 nm or 265 nm, e.g., by using a Hg lamp.

Fig. 6 shows schematically the attachment of the copolymer to the substrate. In Fig. 6, the adhesion points 1 to the substrate 2 surface represent the perfluoro groups of the copolymer. The points shown as being not attached to the surface represent hydrophilic groups, such as polyvinylpyrrolidone units. The covalent binding of the copolymer to the surface (after UV radiation) can be analyzed, for example, by FT-IR through the disappearance of the azide peak, said peak appearing at 2134 $cm^{-1}$ in the spectrum before UV radiation.

In a preferred embodiment, the substrate is dipped into the coating composition and drawn out from the coating composition at a defined velocity to obtain a defined coating thickness. The film thickness of the coating can be determined by the Landau-Levich equation:

$$h = 0.94 \frac{(v_0 \eta)^{2/3}}{(g\rho)^{1/2} \gamma_{LV}^{1/6}}$$

wherein v is the velocity, $\eta$ is the viscosity, $\rho$ is the density, and $\gamma$ is the surface tension. The film thickness can be e.g., 2 $\mu$m.

The substrate of the vascular medical product can comprise a polymer selected from polyamide, for example, polyamide 11 or polyamide 12; polyetherblockamides; thermoplastic polyurethanes (TPU); polyester, for example, PET, or polyester-based elastomers; polyolefins, for example, HDPE, LDPE, PP or olefin-based co-polymers; vinylpolymers, for example, polystyrene; polysiloxanes; and combinations of one or more thereof.

In preferred embodiments of the invention, the vascular medical product is a medical product or part thereof, which is intended to be implanted or introduced into vessels, particularly in the fields of electrophysiology, heart-rhythm management, neurostimulation, vascular and endovascular intervention, intervention in and at the heart. Particularly preferred vascular medical products are selected from coronary or peripheral catheters or parts thereof, CRM devices or parts, balloon parts, or pacemaker electrodes.

In another preferred embodiment, the coating method of the invention is carried out in automated manner.

The present invention further provides a coated vascular medical product that is obtained by the coating according to the invention or the coating method described above.

The coatings obtained by the invention exhibit low friction to friction partners, e.g., blood vessels, and release of a very low number of particles. The release of particles can be determined by a particle counter using e.g., a simulated use model according to ASTM F2394-07(2017), X.2.4. In an exemplary embodiment for a catheter with hydrophilic coated particles, a desirable value is less than 2000 particles release, wherein the limit for particles above 10 $\mu$m is 6000 particles. The friction of the coating scan be determined by the friction coefficient COF.

## Claims

1. A method for coating a surface of a vascular medical product with a coating composition, wherein the coating composition comprises:

    a) 0.1 to 10 weight% of polyvinylpyrrolidone having a molecular weight of less than 1,000,000 g/mol,
    b) 0.1 to 10 weight% of polyvinylpyrrolidone having a molecular weight of more than 1,000,000 g/mol,
    c) 0.1 to 5 weight% of a copolymer having at least

        1) repeating units of vinylpyrrolidone, and
        2) at least one perfluorophenyl azide moiety, and

    d) 0 to 1 weight% of a multifunctional UV-sensitive lower-molecular phenyl azide compound having an UV-absorption in the range of 200 to 300 nm,

    wherein the components (a) to (d) are dissolved in 50 to 90 volume% of alcohol, preferably ethanol and/or 2-propanol, and 10 to 50 volume% of water.

2. The method according to claim 1, wherein the copolymeric coating is applied to the surface by dip coating, spray

coating, roller coating, printing, painting, or by means of a doctor blade, doctor roller or Langmuir-Blodgett films.

**3.** The method according to claim 1 or 2, wherein phenyl azide compound d) is a triazide, polyethyleneglycol-based bisazide, diol bisazide, and/or an ionic bisazide.

**4.** The method according to claim 3, wherein phenyl azide compounds have the following structure:

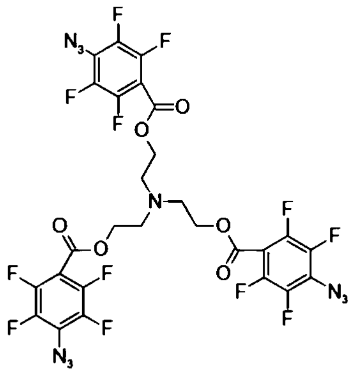
,
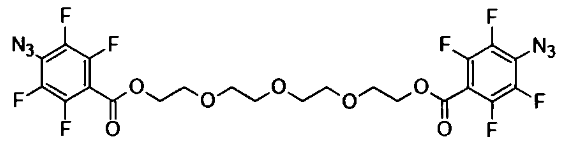
,
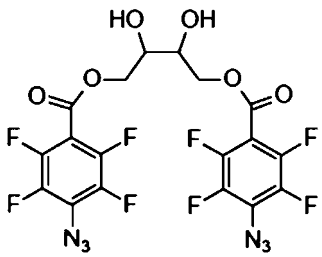
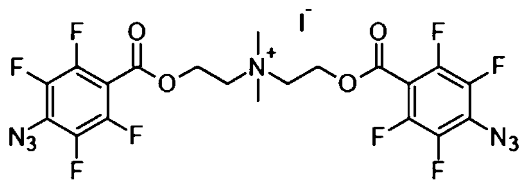
or
.

**5.** The method according to any one of the preceding claims, wherein the surface of the vascular medical product comprises or is made of a polymer selected from polyamide, polyetherblockamides, thermoplastic polyurethanes (TPU), polyester, polyester-based elastomers, thermoplastic elastomers, polyolefins, vinylpolymers, polysiloxanes, and combinations thereof.

**6.** The method according to any one of the preceding claims, wherein the copolymer has the following chemical structure:

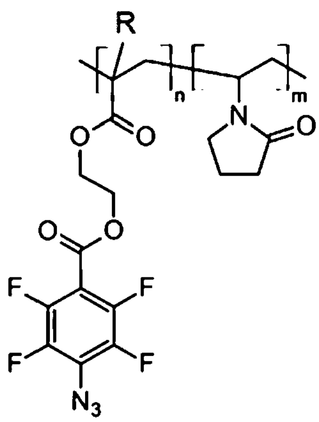
,

wherein R is H or $CH_3$ wherein n is an integer 1 or 2, and m is an integer in the range of from 15 to 35; preferably n is an integer 1 and m is an integer in the range of from 20 to 30; or

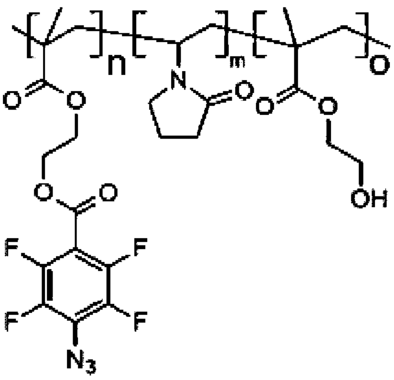

,

wherein n is 1 or 2, m is 15 to 25, and o is 5 to 15; preferably n is 1, m is 20, and o is 10; or

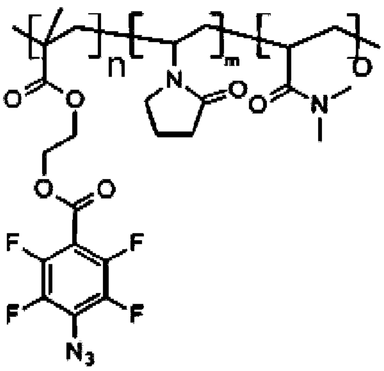

,

wherein n is 1 or 2, m is 15 to 25, and o is 5 to 15; preferably n is 1, m is 20, and o is 1 to 10.

**7.** A vascular medical product, preferably obtained by the method according to any one of claims 1 to 6, comprising a coating composition, wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer having at least

1) repeating units of vinylpyrrolidone, and
2) at least one perfluorophenyl azide moiety, and

d) 0 to 1 weight% of a multifunctional UV-sensitive lower-molecular phenyl azide compound having an UV-absorption in the range of 200 to 300 nm.

**8.** The vascular medical product according to claim 7, wherein the coating composition is bound to a surface of the vascular medical product and comprises at least one of the following copolymers or consists of one of the following copolymers:

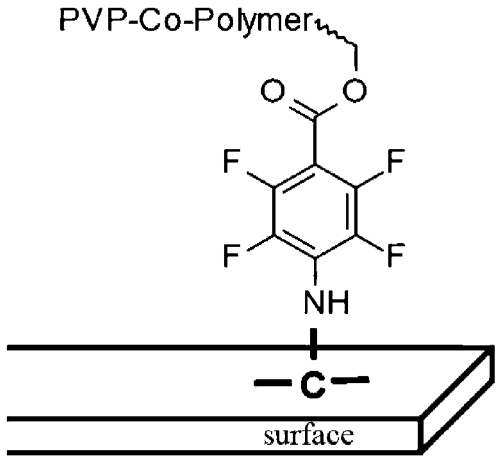

9. The vascular medical product according to claim 7, wherein the coating composition comprises at least one of the following copolymers or consists of one of the following copolymers:

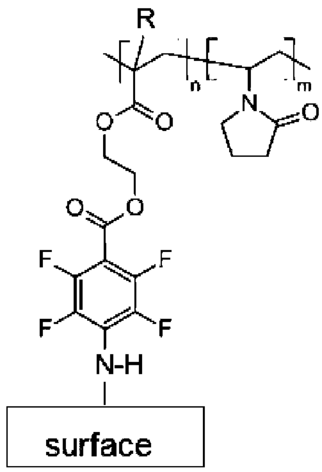

,

wherein R is H or $CH_3$ and wherein n is an integer 1 or 2, and m is an integer in the range of from 15 to 35; preferably n is an integer 1 and m is an integer in the range of from 20 to 30; or

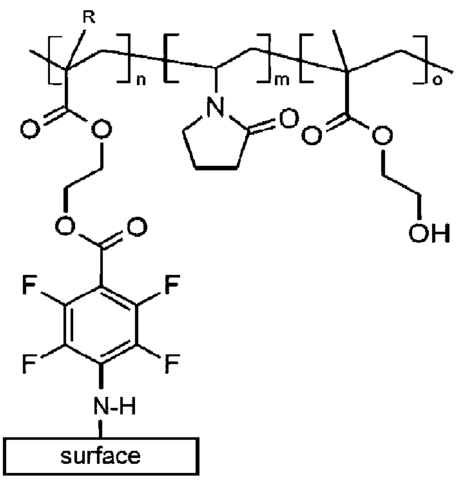

,

wherein R is H or $CH_3$ and wherein n is 1 or 2, m is 15 to 25, and o is 5 to 15; preferably n is 1, m is 20, and o is 10; or

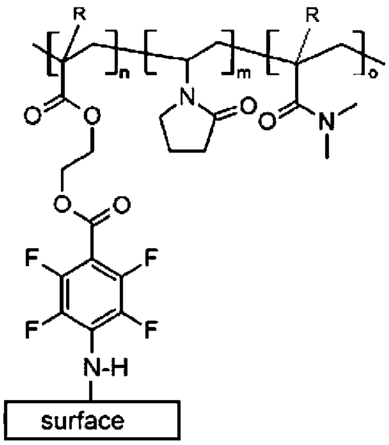

wherein R is H or $CH_3$ and wherein n is 1 or 2, m is 15 to 25, and o is 5 to 15; preferably wherein n is 1, m is 20, and o is 1 to 10.

**10.** Use of a coating composition for coating a surface of a vascular medical product, wherein the coating composition comprises:

a) 0.1 to 10 weight% of polyvinylpyrrolidone having a molecular weight of less than 1,000,000 g/mol,
b) 0.1 to 10 weight% of polyvinylpyrrolidone having a molecular weight of more than 1,000,000 g/mol,
c) 0.1 to 5 weight% of a copolymer having at least

1) repeating units of vinylpyrrolidone, and
2) at least one perfluorophenyl azide moiety, and

d) 0 to 1 weight% of a multifunctional UV-sensitive lower-molecular phenyl azide compound having an UV-absorption, preferably in the range of 200 to 300 nm.

**Patentansprüche**

**1.** Verfahren zum Beschichten einer Oberfläche eines vaskulären Medizinprodukts mit einer Beschichtungszusammensetzung, wobei die Beschichtungszusammensetzung umfasst:

a) 0,1 bis 10 Gew.-% Polyvinylpyrrolidon mit einem Molekulargewicht von weniger als 1.000.000 g/mol,
b) 0,1 bis 10 Gew.-% Polyvinylpyrrolidon mit einem Molekulargewicht von mehr als 1.000.000 g/mol,
c) 0,1 bis 5 Gew.-% eines Copolymers mit mindestens

1) Wiederholungseinheiten aus Vinylpyrrolidon und
2) mindestens eine Perfluorphenylazid-Gruppe, und

d) 0 bis 1 Gew.-% einer multifunktionellen, UV-empfindlichen, niedermolekularen Phenylazidverbindung mit einer UV-Absorption im Bereich von 200 bis 300 nm,

wobei die Komponenten (a) bis (d) in 50 bis 90 Vol.-% Alkohol, vorzugsweise Ethanol und/oder 2-Propanol, und 10 bis 50 Vol.-% Wasser gelöst sind.

**2.** Verfahren nach Anspruch 1, wobei die copolymerische Beschichtung durch Tauchbeschichtung, Sprühbeschichtung, Walzbeschichtung, Drucken, Streichen oder mittels Rakel, Rakelwalze oder Langmuir-Blodgett-Filmen auf die Oberfläche aufgebracht wird.

**3.** Verfahren nach Anspruch 1 oder 2, wobei die Phenylazidverbindung d) ein Triazid, ein Bisazid auf Polyethylenglykolbasis, ein Diolbisazid und/oder ein ionisches Bisazid ist.

**4.** Verfahren nach Anspruch 3, wobei die Phenylazidverbindungen die folgende Struktur aufweisen:

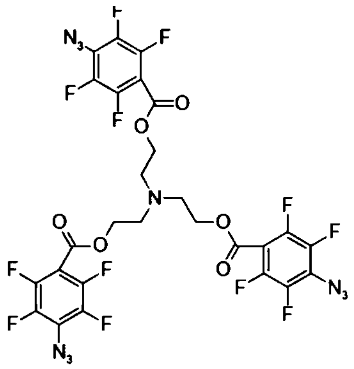

,

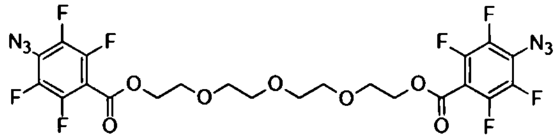

,

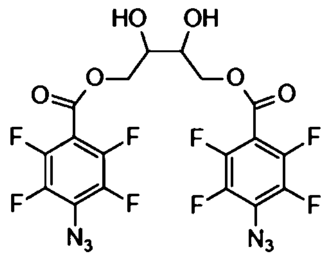

oder

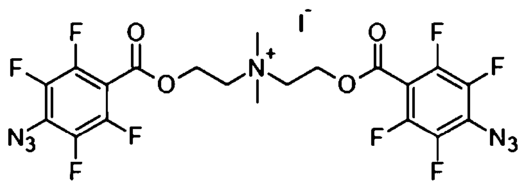

.

5. Verfahren nach einem der vorhergehenden Ansprüche , wobei die Oberfläche des vaskulären Medizinprodukts ein Polymer umfasst oder aus einem Polymer besteht, das aus Polyamid, Polyetherblockamiden, thermoplastischen Polyurethanen (TPU), Polyester, Elastomeren auf Polyesterbasis, thermoplastischen Elastomeren, Polyolefinen, Vinylpolymeren, Polysiloxanen und Kombinationen davon ausgewählt ist.

6. Verfahren gemäß einem der vorhergehenden Ansprüche, wobei das Copolymer die folgende chemische Struktur aufweist:

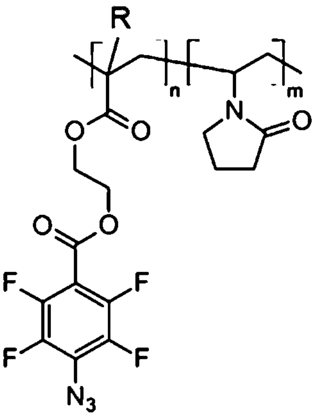

,

wobei R H oder $CH_3$ ist, wobei n eine ganze Zahl von 1 oder 2 ist und m eine ganze Zahl im Bereich von 15 bis 35 ist, vorzugsweise ist n eine ganze Zahl von 1 und m eine ganze Zahl im Bereich von 20 bis 30, oder

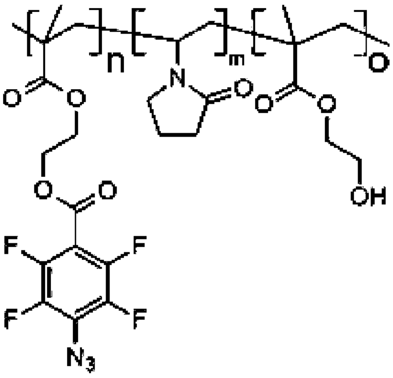

,

wobei n 1 oder 2 ist, m 15 bis 25 ist und o 5 bis 15 ist, vorzugsweise ist n 1, m 20 und o 10, oder

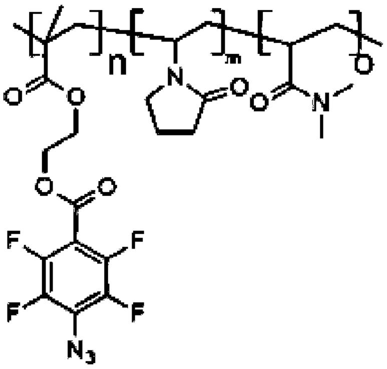

,

wobei n 1 oder 2 ist, m 15 bis 25 ist und o 5 bis 15 ist, vorzugsweise ist n 1, m 20 und o 1 bis 10.

**7.** Ein vaskuläres Medizinprodukt, vorzugsweise erhalten durch das Verfahren gemäß einem der Ansprüche 1 bis 6, umfassend eine Beschichtungszusammensetzung, wobei die Beschichtungszusammensetzung umfasst:

a) 0,1 bis 10 Gew.-% Polyvinylpyrrolidon mit einem Molekulargewicht von weniger als 1.000.000 g/mol,
b) 0,1 bis 10 Gew.-% Polyvinylpyrrolidon mit einem Molekulargewicht von mehr als 1.000.000 g/mol,
c) 0,1 bis 5 Gew.-% eines Copolymers mit mindestens

1) Wiederholungseinheiten aus Vinylpyrrolidon und
2) mindestens eine Perfluorphenylazid-Gruppe, und

d) 0 bis 1 Gew.-% einer multifunktionellen, UV-empfindlichen, niedermolekularen Phenylazidverbindung mit einer UV-Absorption im Bereich von 200 bis 300 nm.

**8.** Das vaskuläre Medizinprodukt nach Anspruch 7, wobei die Beschichtungszusammensetzung an eine Oberfläche des vaskulären Medizinprodukts gebunden ist und mindestens eines der folgenden Copolymere umfasst oder aus einem der folgenden Copolymere besteht:

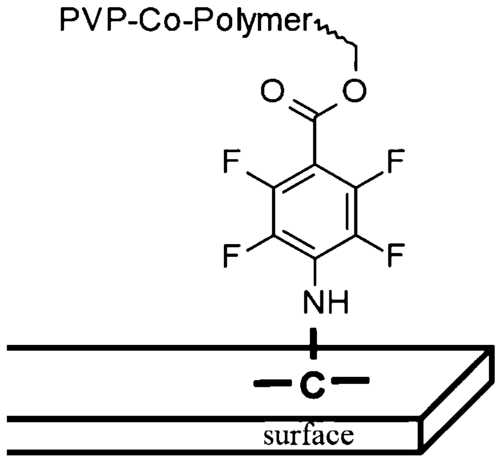

9. Das vaskuläre Medizinprodukt nach Anspruch 7, wobei die Beschichtungszusammensetzung mindestens eines der folgenden Copolymere umfasst oder aus einem der folgenden Copolymere besteht:

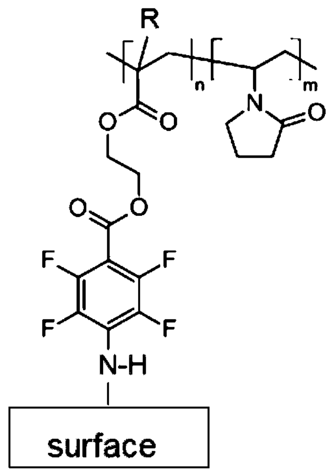

,

wobei R H oder $CH_3$ ist und wobei n eine ganze Zahl von 1 oder 2 ist und m eine ganze Zahl im Bereich von 15 bis 35 ist, vorzugsweise ist n eine ganze Zahl von 1 und m eine ganze Zahl im Bereich von 20 bis 30, oder

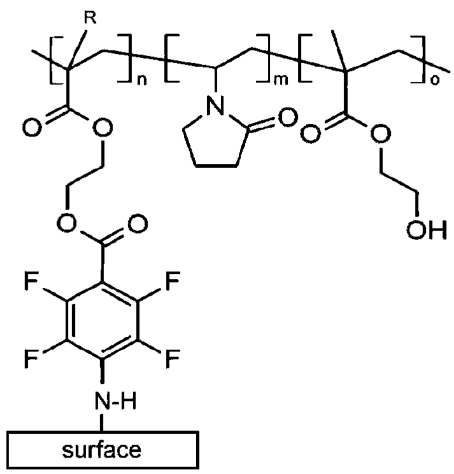

,

wobei R H oder $CH_3$ ist und wobei n 1 oder 2 ist, m 15 bis 25 ist und o 5 bis 15 ist, vorzugsweise ist n 1, m 20 und o 10; oder

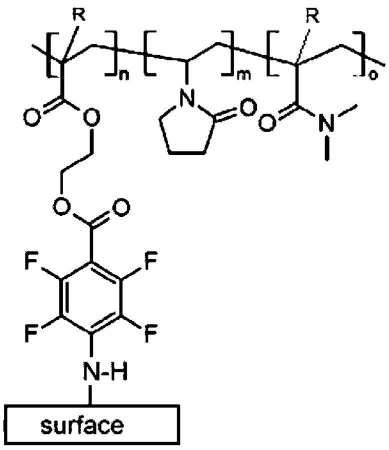

,

wobei R H oder $CH_3$ ist und wobei n 1 oder 2 ist, m 15 bis 25 ist und o 5 bis 15 ist, vorzugsweise wobei n 1 ist, m 20 ist und o 1 bis 10 ist.

**10.** Verwendung einer Beschichtungszusammensetzung zum Beschichten einer Oberfläche eines vaskulären Medizinprodukts, wobei die Beschichtungszusammensetzung umfasst:

a) 0,1 bis 10 Gew.-% Polyvinylpyrrolidon mit einem Molekulargewicht von weniger als 1.000.000 g/mol,
b) 0,1 bis 10 Gew.-% Polyvinylpyrrolidon mit einem Molekulargewicht von mehr als 1.000.000 g/mol,
c) 0,1 bis 5 Gew.-% eines Copolymers mit mindestens

1) Wiederholungseinheiten aus Vinylpyrrolidon und
2) mindestens eine Perfluorphenylazid-Gruppe, und

d) 0 bis 1 Gew.-% einer multifunktionellen, UV-empfindlichen, niedermolekularen Phenylazidverbindung mit einer UV-Absorption, vorzugsweise im Bereich von 200 bis 300 nm.

**Revendications**

**1.** Procédé de revêtement d'une surface d'un produit médical vasculaire avec une composition de revêtement, dans lequel la composition de revêtement comprend :

a) de 0,1 à 10 % en poids de polyvinylpyrrolidone ayant une masse moléculaire inférieure à 1 000 000 g/mol,
b) de 0,1 à 10 % en poids de polyvinylpyrrolidone ayant une masse moléculaire supérieure à 1 000 000 g/mol,
c) de 0,1 à 5 % en poids d'un copolymère comportant au moins

1) des unités répétitives de vinylpyrrolidone, et
2) au moins un groupement perfluorophényl azide, et

d) de 0 à 1 % en poids d'un composé phényl azide multifonctionnel, sensible aux UV, de plus faible masse moléculaire, ayant une absorption UV dans la plage de 200 à 300 nm,

dans lequel les composants (a) à (d) sont dissous dans 50 à 90 % en volume d'alcool, de préférence l'éthanol et/ou le 2-propanol, et 10 à 50 % en volume d'eau.

**2.** Procédé selon la revendication 1, dans lequel le revêtement copolymère est appliqué sur la surface par revêtement par immersion, revêtement par pulvérisation, revêtement au rouleau, impression, peinture, ou au moyen d'une racle, d'un rouleau racleur ou de films de Langmuir-Blodgett.

**3.** Procédé selon la revendication 1 ou 2, dans lequel le composé phényl azide d) est un triazide, un bisazide à base de polyéthylèneglycol, un bisazide diol, et/ou un bisazide ionique.

4. Procédé selon la revendication 3, dans lequel les composés phényl azide ont la structure suivante:

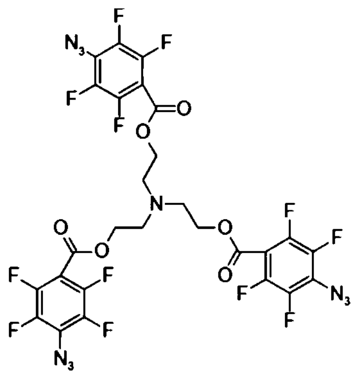
,
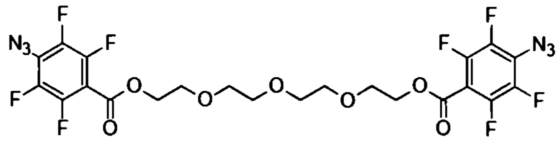
,
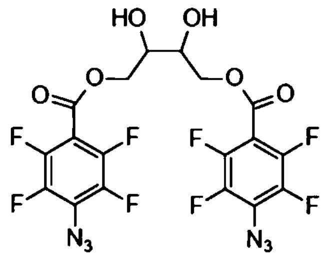
ou
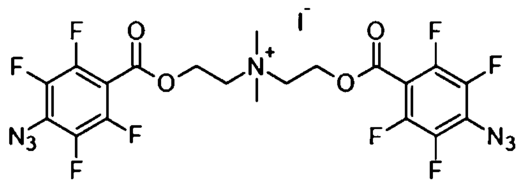
.

5. Procédé selon l'une quelconque des revendications précédentes, dans lequel la surface du produit médical vasculaire comprend ou est constituée d'un polymère choisi parmi le polyamide, les polyéther-block-amides, les polyuréthanes thermoplastiques (TPU), le polyester, les élastomères à base de polyester, les élastomères thermoplastiques, les polyoléfines, les polymères vinyliques, les polysiloxanes, et leurs combinaisons.

6. Procédé selon l'une quelconque des revendications précédentes, dans lequel le copolymère présente la structure chimique suivante:

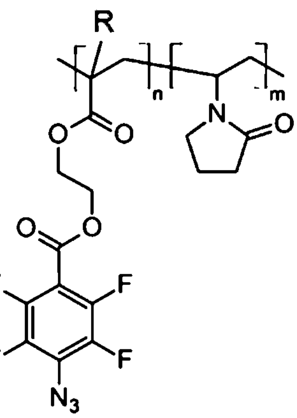

dans laquelle R est H ou $CH_3$, dans laquelle n est un nombre entier 1 ou 2, et m est un nombre entier dans la plage de 15 à 35; de préférence n est un nombre entier 1 et m est un nombre entier dans la plage de 20 à 30; ou

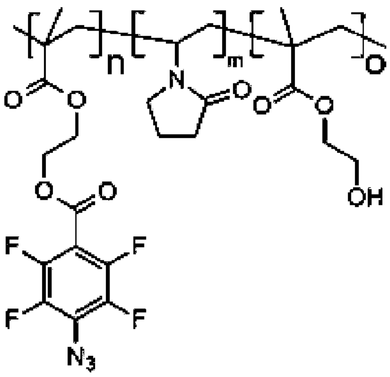

dans laquelle n est 1 ou 2, m est 15 à 25, et o est 5 à 15; de préférence n est 1, m est 20, et o est 10; ou

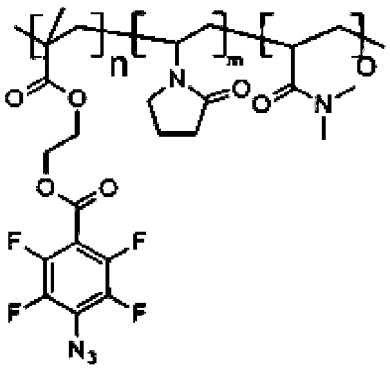

dans laquelle n est 1 ou 2, m est 15 à 25, et o est 5 à 15; de préférence n est 1, m est 20, et o est 1 à 10.

**7.** Produit médical vasculaire, de préférence obtenu par le procédé selon l'une quelconque des revendications 1 à 6, comprenant une composition de revêtement, dans lequel la composition de revêtement comprend:

a) de 0,1 à 10 % en poids de polyvinylpyrrolidone ayant une masse moléculaire inférieure à 1 000 000 g/mol,
b) de 0,1 à 10 % en poids de polyvinylpyrrolidone ayant une masse moléculaire supérieure à 1 000 000 g/mol,
c) de 0,1 à 5 % en poids d'un copolymère comportant au moins

1) des unités répétitives de vinylpyrrolidone, et
2) au moins un groupement perfluorophényl azide, et

d) de 0 à 1 % en poids d'un composé phényl azide multifonctionnel, sensible aux UV, de plus faible masse moléculaire, ayant une absorption UV dans la plage de 200 à 300 nm.

**8.** Produit médical vasculaire selon la revendication 7, dans lequel la composition de revêtement est liée à une surface du produit médical vasculaire et comprend au moins l'un des copolymères suivants ou est constituée de l'un des copolymères suivants :

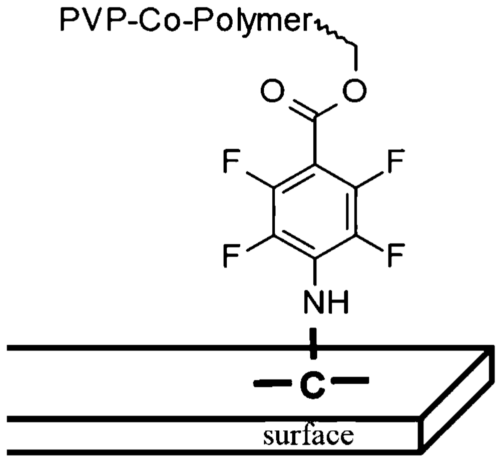

**9.** Produit médical vasculaire selon la revendication 7, dans lequel la composition de revêtement comprend au moins l'un des copolymères suivants ou est constituée de l'un des copolymères suivants:

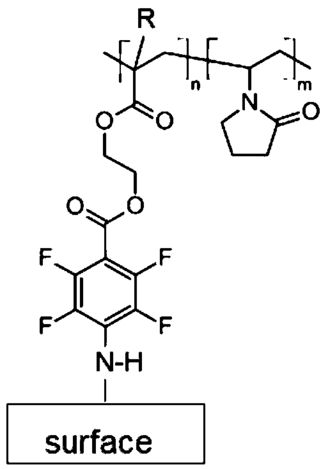

dans laquelle R est H ou $CH_3$ et dans laquelle n est un nombre entier 1 ou 2, et m est un nombre entier dans la plage de 15 à 35; de préférence n est un nombre entier 1 et m est un nombre entier dans la plage de 20 à 30; ou

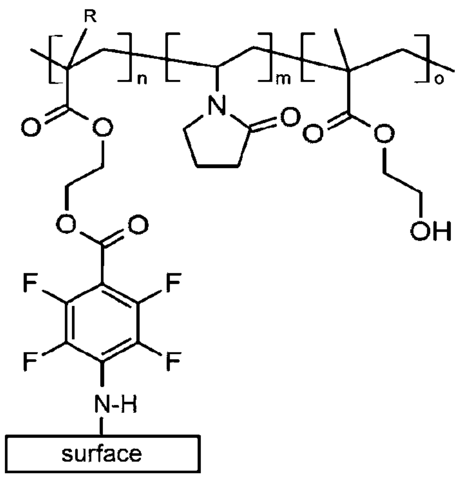

dans laquelle R est H ou $CH_3$ et dans laquelle n est 1 ou 2, m est 15 à 25, et o est 5 à 15; de préférence n est 1, m est 20, et o est 10; ou

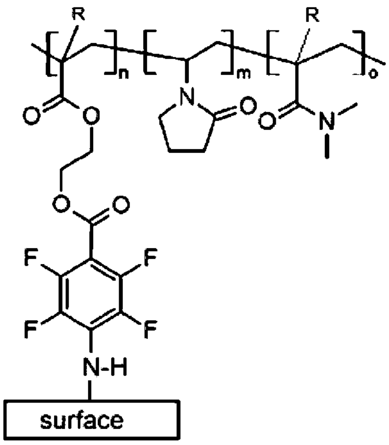

dans laquelle R est H ou $CH_3$ et dans laquelle n est 1 ou 2, m est 15 à 25, et o est 5 à 15; de préférence n est 1, m est 20, et o est 1 à 10.

**10.** Utilisation d'une composition de revêtement pour le revêtement d'une surface d'un produit médical vasculaire, dans laquelle la composition de revêtement comprend :

a) de 0,1 à 10 % en poids de polyvinylpyrrolidone ayant une masse moléculaire inférieure à 1 000 000 g/mol,
b) de 0,1 à 10 % en poids de polyvinylpyrrolidone ayant une masse moléculaire supérieure à 1 000 000 g/mol,
c) de 0,1 à 5 % en poids d'un copolymère comportant au moins

1) des unités répétitives de vinylpyrrolidone, et
2) au moins un groupement perfluorophényl azide, et

d) de 0 à 1 % en poids d'un composé phényl azide multifonctionnel, sensible aux UV, de plus faible masse moléculaire, ayant une absorption UV, de préférence dans la plage de 200 à 300 nm.

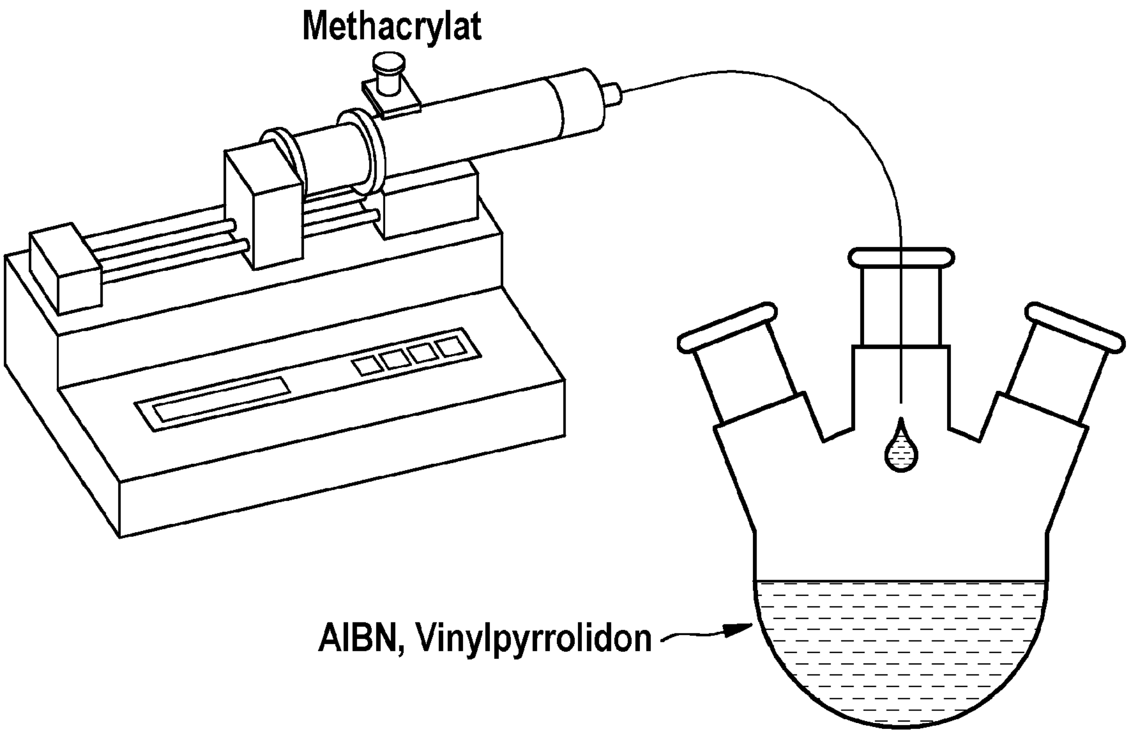
Methacrylat
AIBN, Vinylpyrrolidon

FIG. 1

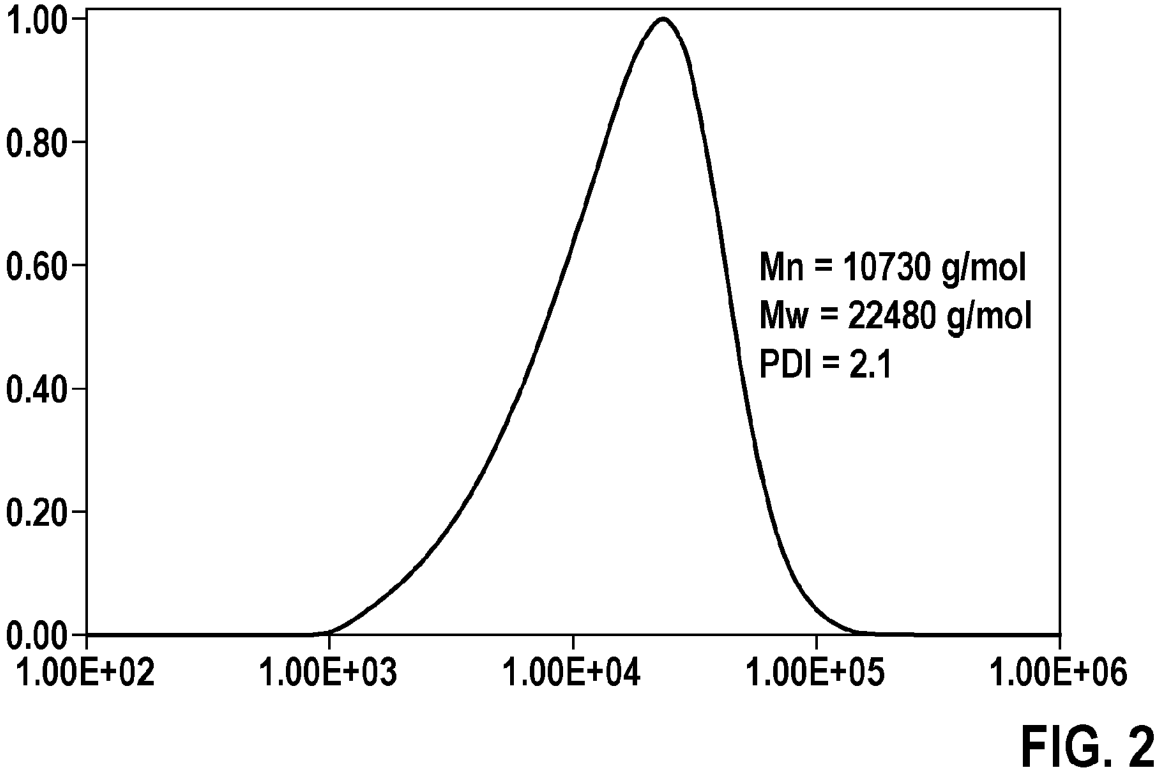
1.00
0.80
0.60
0.40
0.20
0.00
1.00E+02
1.00E+03
1.00E+04
1.00E+05
1.00E+06
Mn = 10730 g/mol
Mw = 22480 g/mol
PDI = 2.1

FIG. 2

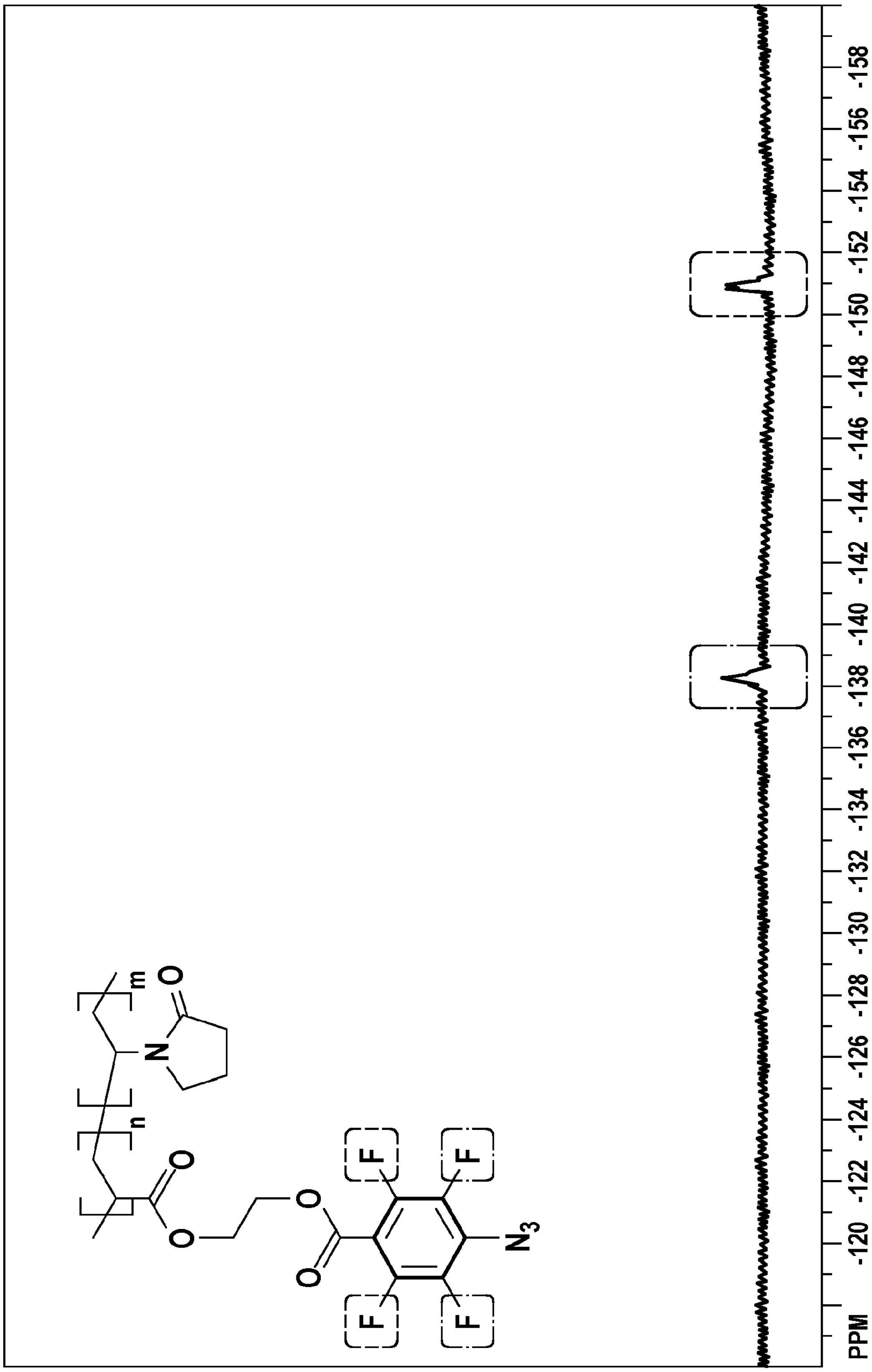
PPM
-120
-122
-124
-126
-128
-130
-132
-134
-136
-138
-140
-142
-144
-146
-148
-150
-152
-154
-156
-158

FIG. 3

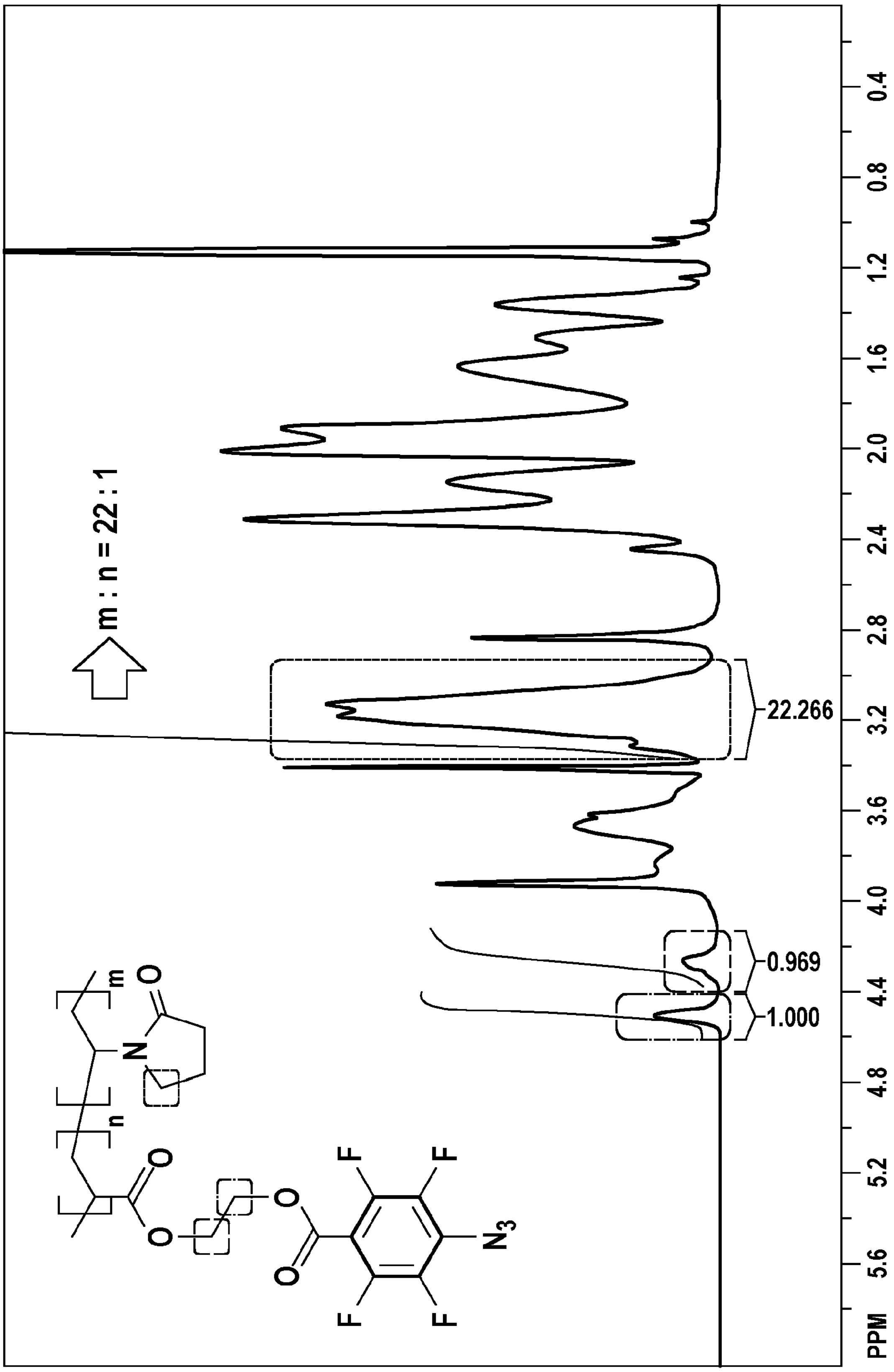


FIG. 4

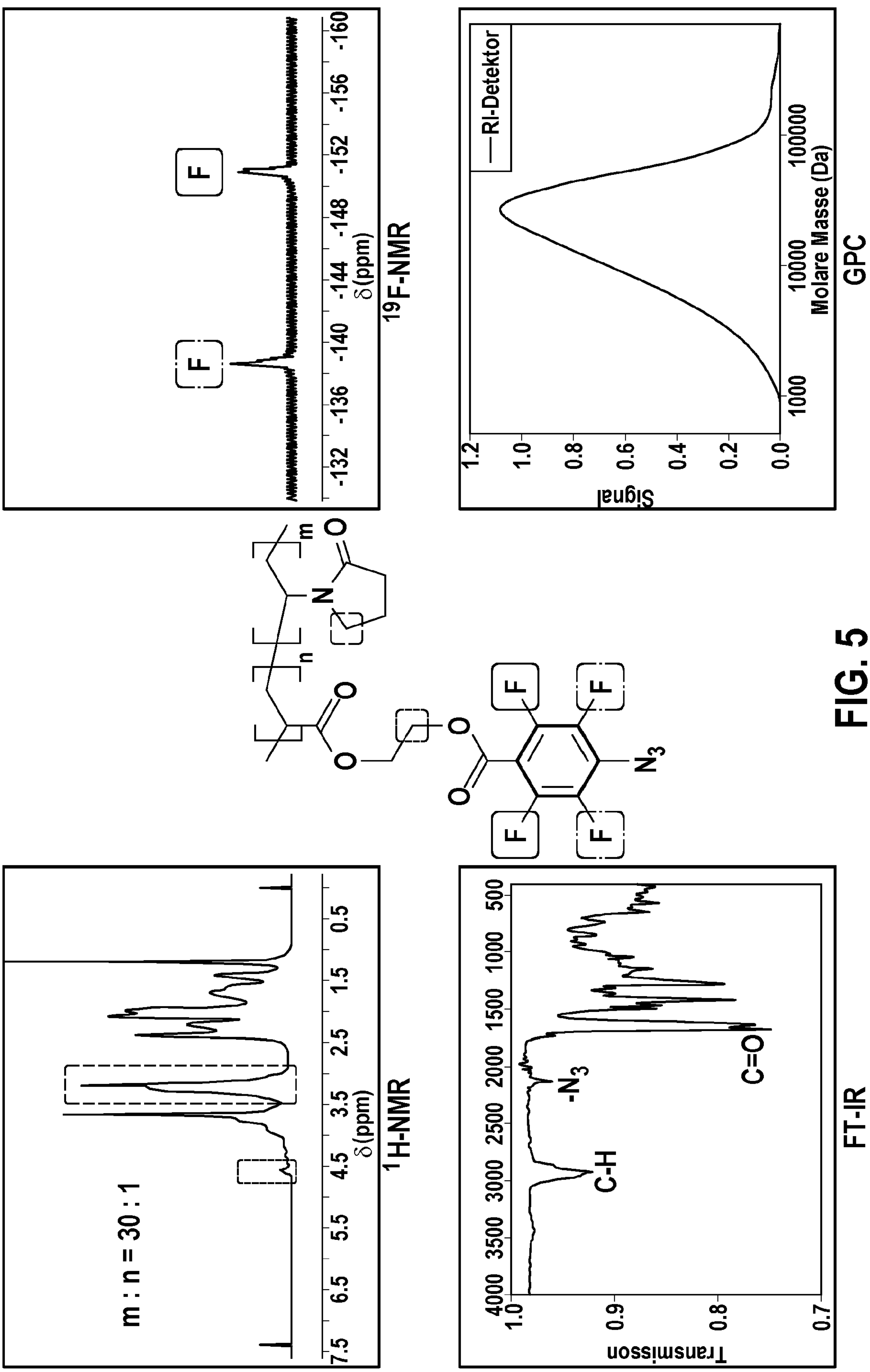
m : n = 30 : 1
7.5 6.5 5.5 4.5 3.5 2.5 1.5 0.5
δ(ppm)
1H-NMR
F
F
-132 -136 -140 -144 -148 -152 -156 -160
δ(ppm)
19F-NMR
Transmisson
1.0 0.9 0.8 0.7
4000 3500 3000 2500 2000 1500 1000 500
C-H
-N3
C=O
FT-IR
Signal
1.2 1.0 0.8 0.6 0.4 0.2 0.0
1000 10000 100000
Molare Masse (Da)
RI-Detektor
GPC

FIG. 5

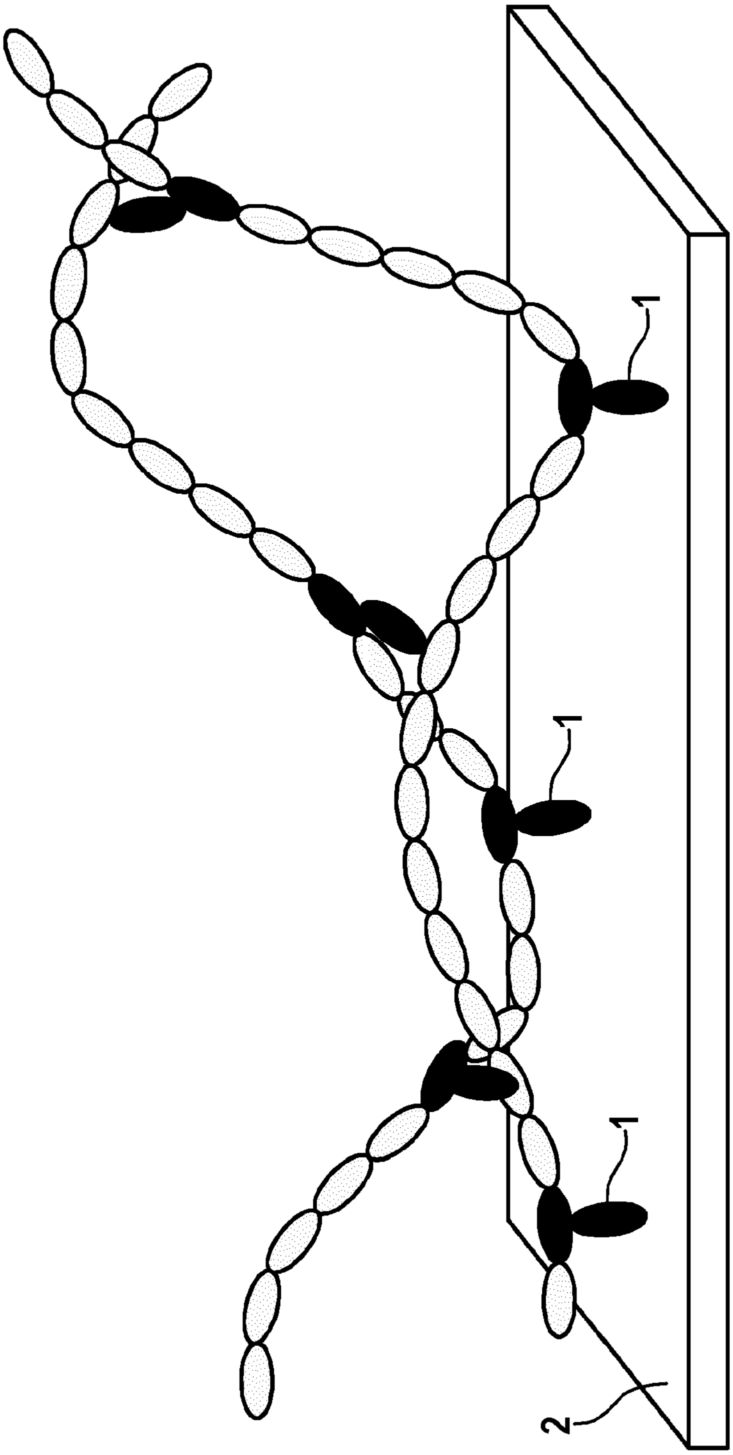


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2020086008 A1


### Non-patent literature cited in the description


- **SIEGMANN, K.** ; **INAUEN, J.** ; **VILLAMAINA, D** ; **WINKLER, M.** Photografting of perfluoroalkanes onto polyethylene surfaces via azide/nitrene chemistry. *Applied Surface Science*, 2017, vol. 396, 672-680
- **SIEGMANN, K.** ; **INAUEN, J.** ; **STERCHI, R** ; **WINKLER, M.** Spectroscopy on photografted polyethylene surfaces using a perfluorophenyl azide: Evidence for covalent attachment. *Surface and Interface Analysis*, 2018, vol. 50 (2), 205-211